# EUROPEAN PATENT SPECIFICATION

(11) **EP 2 126 098 B1**
(45) Date of publication and mention of the grant of the patent: **09.09.2015**
(21) Application number: 07848525.7
(22) Date of filing: 14.12.2007
(51) Int. Cl.: C12N 15/82, A01H 5/00

(54) **SELECTION METHOD**
SELEKTIONSVERFAHREN
PROCÉDÉ DE SÉLECTION

(30) Priority: 15.12.2006 GB 0625076
(43) Date of publication of application: 02.12.2009
(73) Proprietor: Plastid As, 4068 Stavanger (NO); Webber, Philip Michael, London EC4Y 8JD (GB)
(72) Inventor: MOLLER, Simon, Geir, 4036 Stavanger (NO); CHUA, Nam-Hai, New York, NY 10021 (US)
(74) Representative: Webber, Philip Michael
(86) International application number: PCT/GB2007/004782
(87) International publication number: WO 2008/071973

(56) References cited:
- WO-A-01/64023
- WO-A-03/057834
- SCUTT CHARLES P ET AL: "Techniques for the removal of marker genes from transgenic plants." BIOCHIMIE (PARIS), vol. 84, no. 11, November 2002 (2002-11), pages 1119-1126, XP002479652 ISSN: 0300-9084
- ARAGAO FRANCISCO JOSE LIMA ET AL: "Positive, negative and marker-free strategies for transgenic plant selection." BRAZILIAN JOURNAL OF PLANT PHYSIOLOGY, vol. 14, no. 1, 2002, pages 1-10, XP002479653 ISSN: 1677-0420

## Description

The invention relates to a method for producing a transformed plant cell. More particularly, the method involves the transformation of a plant cell with a Transformation Cassette which is targeted to plant plastids and which comprises the selection gene isopentenyl transferase (IPT) and a transgene. After selection for transformed plastids, expression of a recombinase is induced in the plant cell, which leads to the excision of the selection gene from the plastid and the expression of the transgene in the plastid. The invention also provides cells and plants comprising the Transformation Cassette.

The use of genetically modified (GM) food crops in agriculture is rapidly increasing with an approximate £14 billion world market in 2005. There is, however, a growing concern amongst scientists, politicians, regulatory agencies and the general public regarding the risk of the spread of transgenes by pollen from GM crops to other plant species generating so-called "superweeds".

All commercial GM crops used in today's agriculture have engineered nuclear genomes containing transgenes conferring desirable traits such as resistance to disease, insects, harsh environmental conditions and increased vitamin content, flavour, and storage time. However, there are several serious problems with nuclear transgenes. Nuclear transgenes are spread by pollen, are generally expressed at low levels, are often silenced (shut-down), and can only be expressed individually, making biochemical pathway engineering very difficult. One way of resolving the problems associated with nuclear transgenes is to insert transgenes into the plastid genome of plants.

Plastids are organelles unique to plants. Each plant cell contains approximately 100 plastids each of which contains approximately 100 genomes. This in effect means that when a gene is inserted into the plastid genome each plant cell contains approximately 10,000 copies of that gene compared to plant nuclear transformation where each plant cell would have at best 2-3 copies of the gene.

The expression of transgenes in plastids has numerous advantages over nuclear gene expression: (i) transgenes are not spread by pollen; (ii) proteins are expressed to high levels (up to 47% of total cellular protein); (iii) "toxic" effects of proteins are reduced due to plastid containment; (iv) the simultaneous expression of several genes allows for biochemical pathway engineering; and (v) gene silencing is eliminated. Plastid genetic engineering has clear fundamental and applied applications in that potentially any protein can be produced to high levels with little environmental risk opening up the possibility of producing edible vaccines, biopharmaceuticals and products of agronomical value.

Currently, the only reliable selection systems that are used to select for plastids, which have been transformed with transgenes, are based on the use of antibiotics such as spectinomycin. A disadvantage of such selection systems is that they will also select for spontaneous ribosomal mutants which are resistant to spectinomycin. This is a large problem, given the time-scales involved.

The invention is based on a selection and regeneration system for plastid transformation based on the over-expression of the isopentenyl transferase (IPT) gene (cytokinin biosynthesis) in plastids. This system allows for the direct selection of cells containing transformed plastids on media lacking cytokinin due to cytokinin production within plastids. The system therefore provides an antibiotics-free selection and regeneration system which will overcome problems with spontaneous spectinomycin mutants and concerns regarding the use of antibiotic resistance genes in GMOs.

Whilst IPT has previously been used as a selectable marker in plant nuclear transformation (e.g. EP 1 069 855 A), its used in plastid transformation has not previously been suggested. The person skilled in the art will be aware of the fact that plastids are semi-autonomous organelles within plant cells with their own genomes and metabolism. Hence the criteria for choosing plastid-selection genes are distinct from those for choosing genome-selection genes.

Due to the fact that over-expression of plant-hormone biosynthetic polypeptides in adult plants leads to impaired development, the plant-hormone biosynthetic gene is removed after selection and initial regeneration has occurred. The transgene in question (expressing the polypeptide of interest) is only activated after the removal of the plant-hormone biosynthetic gene, so that any adverse effects due to transgene expression during selection and regeneration are also eliminated.

In one embodiment, the invention provides a method for producing a transformed plant cell, the method comprising the step:
(i) transforming the plant cell with a genetic construct,
   wherein the genetic construct comprises first and second homologous recombination elements flanking a Transformation Cassette, wherein the nucleotide sequences of the first and second homologous recombination elements are selected such that the Transformation Cassette is specifically targeted to one or more selected plastids and wherein the first and second homologous recombination elements direct the integration of the Transformation Cassette into the genome of the one or more selected plastids which are present in the plant cell,
   wherein the Transformation Cassette comprises:
   (a) a first promoter which is operable in said plant cell,
   (b) an Excision Cassette,
   (c) one or more transgenes,
   (d) a first terminator element,
   wherein the Excision Cassette comprises:
   (b1) a first site-specific recombination element,
   (b2) an optional second promoter
   (b3) a nucleotide sequence encoding a plant-hormone biosynthetic polypeptide wherein the plant-hormone biosynthetic polypeptide is IPT (isopentenyl transferase),
   (b4) a second terminator element,
   (b5) a second site-specific recombination element,
   wherein the first and second site-specific recombination elements are capable of being recognised by a recombinase, and wherein the method additionally comprises the step:
(ii) selecting for transformed plant cells on media which is lacking cytokinin. In other embodiments, the method additionally comprises the step:
(iii) expressing a recombinase in the plant cell, wherein the recombinase is one which recognises the first and second site-specific recombination elements.

The method of the invention is suitable for all plants that can be transformed and regenerated. The plant may be a monocot or dicot.

Examples of suitable plants are cereals (rice, wheat, barley, oats, sorghum, corn), legumes (alfalfa, lentils, peanut, pea, soybean), oil crops (palm, sunflower, coconut, canola, olive), cash crops (cotton, sugar cane, cassava), vegetable crops (potato, tomato, carrot, sweet potato, sugar-beet, squash, cucumber, lettuce, broccoli, cauliflower, snap bean, cabbage, celery, onion, garlic), fruits/trees and nuts (banana, grape cantaloupe, muskmelon, watermelon, strawberry, orange, apple, mango, avocado, peach, grapefruit, pineapple, maple, almond), beverages (coffee, tea, cocoa), and timber trees (oak, black walnut, sycamore). Preferably, the plant is tobacco or lettuce.

The plant cells which are being transformed may be used in any convenient form, for example, as individual cells, groups of cells, in dissociated form or undissociated form, or as part of a plant tissue or plant part. Preferably, the cells are present in leaves that are removed from intact plants. It is preferable to use actively-growing leaves.

The term "plastid" is intended to cover all organelles which are found in the cytoplasm of eukaryotic plants, which contain DNA, which are bounded by a double membrane, and develop from a common type, i.e. a proplastid. Plastids may contain pigments and/or storage materials.

Examples of plastids include chloroplasts, leucoplasts, amyloplasts, etioplasts, chromoplasts, elaioplasts and gerontoplasts.

Preferably, the plastid is a green plastid, most preferably a chloroplast.

As used herein, the term "genetic construct" refers to a nucleic acid molecule comprising the specified elements and Cassettes. The genetic construct may, for example, be in the form of a vector or a plasmid. It may also contain other elements which enable its handling and reproduction, such as an origin of replication, selection elements, and multiple cloning sites. Generally, the genetic construct will be a double-stranded nucleic acid molecule, preferably a dsDNA molecule.

The first and second homologous recombination elements are ones that are capable of directing the integration of the Transformation Cassette into the genome of at least one plastid which is present in the plant cell.

Upon transformation of the genetic construct into the plant cell, the first and second homologous recombination elements recombine with corresponding sequences in the genome of the selected plastid or plastids, resulting in the insertion of the Transformation Cassette into the genome of the selected plastid or plastids.

The nucleotide sequences of the homologous recombination elements are selected such that the Transformation Cassette is specifically targeted to one or more selected plastids. In particular, the nucleotide sequences of the homologous recombination elements are selected such that no or essentially no Transformation Cassettes become integrated into the nuclear genome of the plant. This may be done by avoiding sequences which are present in the nuclear genome of the plant. The skilled person will readily be able to detect whether a specific sequence is or is not present in the nuclear genome by standard means, for example, by Southern Blotting of the nuclear genome with a labelled sequence probe or by sequence analysis.

Apart from the above, any sequences can be used from the plastid genome as long as the selected insertion site is not lethal to the cell, i.e. it does not result in the death of the cell. Preferably, the insertion sites are not in coding regions of plastid genes.

The orientation of the sequences of the first and second homologous recombination elements should be the same as the orientation in the plastid genome to allow for efficient homologous recombination.

In order to target the Transformation Cassette to the plastid genome, the nucleotide sequences of the first and second homologous recombination elements must be identical or substantially identical to sequences in the genome of the selected plant plastid.

In the context of the present invention, the term "substantially identical" means that the nucleotide sequences of the first and second homologous recombination sequences are independently more than 95%, preferably more than 98% or more than 99% and particularly preferably 100% identical to sequences which are present in the plastid to be transformed. Percentage sequence identities may be determined using the Clustal method of alignment with default parameters, e.g. KTUPLE 1, GAP PENALTY=3, WINDOW=5 and DIAGONALS SAVED =5.

Similarly, the nucleotide sequences of the first and second homologous recombination elements should preferably not be identical or substantially identical to sequences in the nuclear genome of the selected plant. In this context, the term "substantially identical" means that the nucleotide sequences of the first and second homologous recombination sequences are independently less than 50%, more preferably less than 70% or less than 90% identical to sequences which are present in the nuclear genome of the plant to be transformed. Percentage sequence identities may be determined using the Clustal method of alignment with default parameters, e.g. KTUPLE 1, GAP PENALTY=3, WINDOW=5 and DIAGONALS SAVED=5.

Preferably, the lengths of first and second homologous recombination sequences will independently be 50-1500 nucleotides each, preferably about 150, about 1000 or about 1200 nucleotides in length.

The distance between the first and second homologous recombination sequences in the plastid genome may be 0-4000 nucleotides or more. Preferably, the distance is about 1-100, 100-500, 500-1000 or 1000-3000 nucleotides.

The total length of the genetic elements which are present between the first and second homologous recombination (i.e. genetic elements (a)-(d)) is preferably less than 4000 nucleotides.

Preferably, the first homologous recombination sequence is nucleotides 104091-105380 of the *Nicotiana tabacum* (accession no. Z00044) chloroplast genome DNA; and/or preferably, the second homologous recombination sequence is nucleotides 105381-106370 of the *Nicotiana tabacum* (accession no. Z00044) chloroplast genome DNA.

In yet other embodiments, the first homologous recombination sequence is preferably nucleotides 102925-101857 of the *Nicotiana tabacum* (accession no: Z00044) choloroplast genome DNA; and/or the second homologous recombination sequence is nucleotides 100933-100130 of the *Nicotiana tabacum* (accession no. Z00044) choloroplast genome DNA.

The Transformation Cassette promoter (a) must be one that is operable in the selected plant plastid. The promoter is one which is capable of initiating transcription of the transgene once the Excision Cassette has been excised; and of initiating the transcription of the nucleotide sequence encoding a plant-hormone biosynthetic polypeptide, in cases where the Excision Cassette does not contain its own promoter. The promoter might, for example, be one derived from a plant or bacterial gene. Preferably, the promoter is plant specific.

Examples of suitable promoters include PsbA, RbcL and Prrn promoters.

Preferably, the promoter is a Prrn promoter (e.g. Plastidic ribosomal RNA (rrn) operon promoter (nt 59034-59303, accession Z00044 *Nicotiana tabacum* chloroplast genome DNA).

In some embodiments, the promoter is an inducible promoter. This allows inducible, controlled expression of the selection gene(s). For example, the inducible promoter may be inducible by IPTG, e.g. the PrrnL promoter.

Alternatively, the promoter is a high-expression level promoter.

The Excision Cassette comprises a first site-specific recombination element; optionally, a second promoter; a nucleotide sequence encoding a plant-hormone biosynthetic polypeptide; a terminator sequence; and a second site-specific recombination element.

The first and second site-specific recombination elements are sequences of nucleotides which are capable of being recognised and/or bound by the site-specific recombinase which is produced by a recombinase. The site-specific recombination elements must flank the genetic elements in the Excision Cassette, e.g. elements (b2) (if used), (b3), (b4), any other desired elements. The sequences of the first and second recombination elements will be identical or substantially identical to each other; and will be in the same orientation relative to each other (e.g. both 5'-3' or both 3'-5').

When the recombinase is Cre, the site-specific recombination sequences are preferably lox sequences. Site-specific lox recombination sites are 34 bp sequences; these act as binding sites for the Cre recombinase polypeptide. Wild-type lox sequences are preferred (Zuo J, Niu QW, Møller SG, Chua NH (2001) Chemical-regulated, site-specific DNA excision in transgenic plants. Nat. Biotechnol. 19, 157-161.)

The Excision Cassette promoter, when present, must be one that is operable in the selected plant plastid. The promoter is one which is capable of initiating transcription of the plant-hormone biosynthetic gene. The promoter might be one derived from a plant or bacterial gene. Preferably, the promoter is plant specific. Examples of suitable promoters include PsbA, RbcL and Prrn promoters. Preferably, the promoter is a Prrn promoter (e.g. Plastidic ribosomal RNA (rrn) operon promoter (nt 59034-59303, accession Z00044 *Nicotiana tabacum* chloroplast genome DNA).

The plant-hormone biosynthetic polypeptide acts as a selection marker, allowing the selection of plant cells which have been transformed with the Transformation Cassette.

The plant-hormone biosynthetic polypeptide is IPT (isopentenyl transferase) which is an enzyme involved in cytokinin biosynthesis. The IPT nucleotide sequence may be from any suitable source. Due to codon usage, bacterial IPT genes are preferred, because nuclear genes may not be expressed to maximum levels in chloroplasts.

Preferably, the IPT nucleotide sequence is from *Agrobacterium tumefaciens.*

The Excision Cassette terminator prevents the premature expression of the transgene(s) prior to the excision of the Excision Cassette. Any terminator can be used for this provided that it is recognised in the plant cell being transformed. The terminator may be a plant terminator or a bacterial terminator, *inter alia.*

Examples of suitable terminators include those of rrn, psbA, rbcL and T7.

The preferred terminator is a TrbcL terminator (e.g. Ribulose 1,5-Bisphosphate Carboxylase/Oxygenase polyA addition sequence (nt 102539-102685, accession Z00044 *Nicotiana tabacum* chloroplast genome DNA).

In some embodiments of the invention, the promoter and terminator used in the Excision Cassette do not both originate from the same plastid gene.
In the context of the present invention, the term "transgene" (i.e. element (c)) is used to refer to a nucleic acid molecule which is being introduced into the genome of the plastid. The transgene may, for example, be a genomic DNA, cDNA or synthetic nucleic acid molecule coding for a peptide or polypeptide; a nucleic acid molecule encoding a RNA, tRNA or ribozyme; or any other nucleic acid molecule.

Examples of transgenes include those coding for antibodies, antibiotics, herbicides, vaccine antigens, enzymes, enzyme inhibitors and design peptides.

Single or multiple antigens may be produced from viridae, bacteria, fungi or other pathogens. The antigens may be expressed as single units or as multiple units of several antigens, e.g. for broad-spectrum vaccine development.

Enzymes may be produced for use in cosmetics (e.g. superoxide dismutase, peroxidase, etc.). Enzymes may also be produced for use in detergent compositions.

The invention particularly relates to the production of proteins/enzymes with specific activities, for example, immunostimulants to boost immune responses, such as interferons; and growth factors, e.g. transforming growth factor-beta (TGF-beta), bone morphogenic protein (BMP), neurotrophins (NGF, BDNF, NT3), fibroblast growth factor (FGF), proteolytic enzymes (papain, bromelain), and food supplement enzymes (protease, lipase, amylase, cellulase).

The invention also relates to the production or overexpression of proteins/enzymes in plastids that make the plants more resistant to biotic and abiotic stresse, such as salts and metals. Examples of this include the generation of transplastomic plants that chelate iron (Fe) for mopping up excess metal in agriculturally important areas for future planting.

The invention further relates to the use of transgenes encoding polypeptides which modify fatty acid biosynthesis in plastids.

One or more transgenes may be inserted in the Transformation Cassette. Preferably, the transgene sequences are contiguous.

The transgene sequence may additionally encode a protein purification tag fused to the polypeptide of interest. Examples of protein purification tags include the N-terminal influenza haemagglutinin-HA-epitope (HA) and a sequence of six histidine amino acids (HIS6).

Once the Excision Cassette has been excised, the first promoter is capable of driving the expression of the transgene, leading to the accumulation of the product of the transgene in the plastid. The product of the transgene may be purified or isolated from the plant cell by any suitable means.

The Transformation Cassette terminator (genetic element (d)) terminates the expression of the transgene(s). Any terminator can be used for this provided that it is recognised in the plant cell being transformed. The terminator may be a plant terminator or a bacterial terminator, *inter alia.*

Examples of suitable terminators include TrbcL or TspbA polyA addition sequences.

The preferred terminator is the *psbA* polyA addition sequence.

In the Transformation Cassette, the elements are preferably operably linked in the order (a), (b), (c), (d).

In the Excision Cassette, the first and second site-specific recombination elements must flank the optional promoter (when present), the nucleotide sequence encoding a plant-hormone biosynthetic polypeptide and the terminator element.

In some embodiments of the invention, the nucleotide sequence encoding a plant-hormone biosynthetic polypeptide and the terminator element will be downstream (i.e. 3') to the promoter of the Transformation Cassette and hence the latter promoter will be capable of driving expression of the plant-hormone biosynthetic polypeptide.

Thus in some embodiments of the invention, the Transformation Cassette comprises:
(a) a first promoter which is operable in said plant cell,
(b) an Excision Cassette comprising:
   (b1) a first site-specific recombination element,
   (b3) a nucleotide sequence encoding a plant-hormone biosynthetic polypeptide, wherein the polypeptide is IPT,
   (b4) a second terminator element,
   (b5) a second site-specific recombination element,
   wherein the first and second site-specific recombination elements are capable of being recognised by a recombinase;
(c) one or more transgenes,
(d) a first terminator element,
operably linked in the order specified above in a 5'-3' direction.

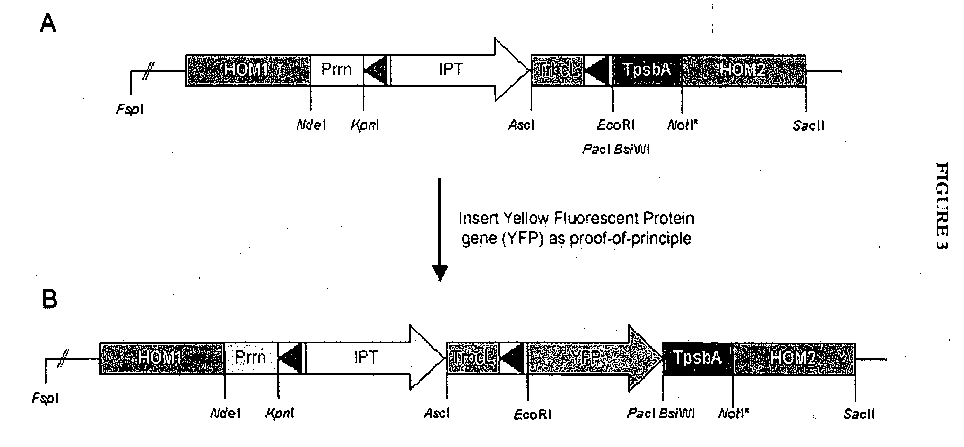

In this embodiment of the invention, the first promoter is capable of driving expression of the nucleotide sequence encoding a plant-hormone biosynthetic polypeptide (for example, as shown in Figures 3-4). After the removal of the Excision Cassette, the first promoter drives expression of the transgene(s).

In other embodiments of the invention, the Excision Cassette will be in the reverse orientation compared to the first promoter, transgene(s) and first terminator element. In this embodiment, the expressed parts of the Excision Cassette will be present in the nucleotide strand which is complementary to that which codes for the first promoter, transgene(s) and first terminator element, and in the reverse direction.

In such embodiments, the Excision Cassette will comprise a second promoter, capable of driving the expression of the nucleotide sequence encoding the plant-hormone biosynthetic polypeptide.

In this embodiment of the invention, the Transformation Cassette comprises:
(a) a first promoter which is operable in said plant cell,
(b) an Excision Cassette,
(c) one or more transgenes,
(d) a first terminator element,
wherein (a), (b), (c) and (d) are operably linked in the order specified above in a 5'-3' direction,
wherein the Excision Cassette comprises:
(b1) a first site-specific recombination element,
(b2) a second promoter,
(b3) a nucleotide sequence encoding a plant-hormone biosynthetic polypeptide, wherein the polypeptide is IPT,
(b4) a second terminator element,
(b5) a second site-specific recombination element,
wherein the first and second site-specific recombination elements are capable of being recognised by a recombinase, and
wherein the parts of the Excision Cassette are operably linked and wherein the Excision Cassette is in reverse orientation compared to (a), (c) and (d).

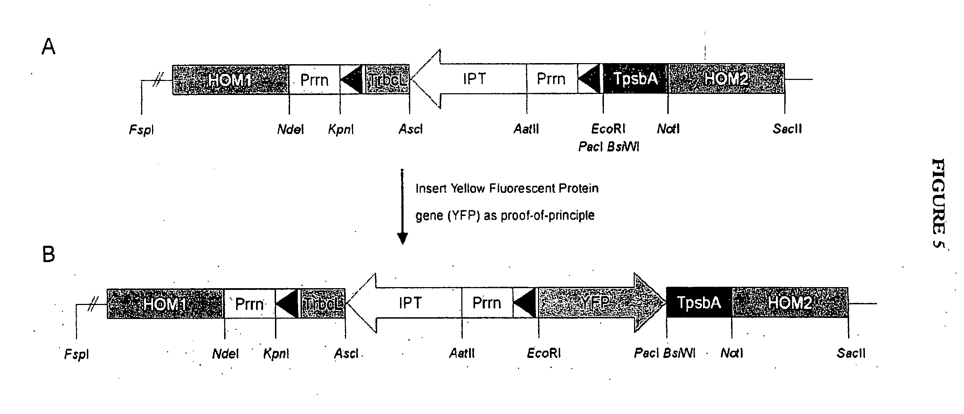

In this embodiment, the second promoter drives expression of the nucleotide sequence encoding a plant-hormone biosynthetic polypeptide (for example, as shown in Figures 5-6). After the removal of the Excision Cassette, the first promoter drives expression of the transgene(s).

The Transformation Cassette is not restricted to the parts (a)-(d) specified herein. It may, for example, additionally comprise a 5'-UTR to increase the expression level of the transgene(s).

In some embodiments of the invention, the Transformation Cassette additionally comprises a second selectable marker gene, e.g. an antibiotic resistance gene, preferably a nucleotide sequence encoding spectinomycin adenyltransferase (e.g. the aadA gene). This polypeptide confers resistance to the antibiotic spectinomycin. The nucleotide sequence enoding spectinomycin adenyltransferase may be placed downstream of one of the promoters in the Transformation Cassette. It may; for example, be placed downstream and operably linked to the first promoter; or downstream and operably linked to the nucleotide sequence encoding a plant-hormone biosynthetic polypeptide (e.g. IPT); or upstream and operably linked to the nucleotide sequence encoding a plant-hormone biosynthetic polypeptide (e.g. IPT).

In yet other embodiments, the Transformation Cassette excludes a second selectable marker gene and/or excludes a nucleotide sequence which confers resistance to an antibiotic.

In some embodiments, the Transformation Cassette additionally comprises the LacI gene, preferably under control of an appropriate promoter (e.g. T7 or Tt7), in order to allow further monitoring of the location of Cassette insertion.

After successful delivery of the Transformation Cassette to the plastid(s), transformed cells are selected on media lacking the plant-hormone cytokinin. In general, plants are regenerated by adding cytokinin and auxin. Because the transformed plastids will produce IPT and therefore cytokinin, plants can be selected and regenerated in the presence of auxin only. The cells for selection will preferably be leaf cells.

In some embodiments of the invention, the method additionally comprises the step:
(iii) expressing a recombinase in the plant cell, wherein the recombinase is one which recognises the first and second site-specific recombination elements.

The recombinase is a site-specific recombinase. A site-specific recombinase is a polypeptide which is capable of binding to site-specific recombination elements and inducing a cross-over event in the nucleic acid molecule in the vicinity of the site-specific recombination elements. In the present invention, the expression of the recombinase leads to the excision of the Excision Cassette from the plastid genome.

In the context of the present invention, the recombinase is one which is capable of binding to the first and second site-specific recombination elements which are present in the Excision Cassette, leading to the excision of the Excision Cassette in a standard manner.

Examples of site-specific recombination elements/site-specific recombinases include Cre-lox, the FLP-FRP system from *Saccharomyces cerevisae* (O'Gorman S, Fox DT, Wahl GM. (1991) Recombinase-mediated gene activation and site-specific integration in mammalian cells. Science. 25, 1351-1555.), the GIN/gix system from bacteriophage Mu (Maeser S and Kahmann R. (1991) The Gin recombinase of phage Mu can catalyse site-specific recombination in plant protoplasts. Mol Gen Genet. 230, 170-176.) or the R/RS system from *Zygosaccharomyces rouxii* (Onouchi H, Yokoi K, Machida C, Matsuzaki H, Oshima Y, Matsuoka K, Nakamura K, Machida Y. (1991) Operation of an efficient site-specific recombination system of Zygosaccharomyces rouxii in tobacco cells. Nucleic Acids Res. 19, 6373-6378.).

The nucleotide sequence which codes for the recombinase may comprise an intron, preferably a plant-specific intron. The presence of such an intron will suppress the expression of the recombinase polypeptide in prokaryotes, for example bacteria.

The preferred recombination site is lox in combination with the recombinase Cre. Preferably, the recombinase sequence used is a cDNA sequence encoding a Cre polypeptide.

Preferably, all or substantially all of the Excision Cassettes are excised from the plastid genome by the recombinase. The skilled person will understand, however, that some or all of the sequences of one or more recombination elements might remain in the plastid genome.

The recombinase may be expressed in the cell by any suitable means.

In some embodiments of the invention, the plant cell is one which already comprises an expressible construct which is integrated into the nuclear genome, wherein the expressible construct comprises a nucleotide sequence encoding a plastid-targeting transit peptide and a recombinase (operably linked, i.e. in frame). The expressible construct might, for example, have been introduced into the nuclear genome by homologous recombination. In such cases, the recombinase must be under the control of an inducible promoter. Such an inducible promoter may have been introduced with the construct or the construct may have been integrated adjacent to an endogenous inducible promoter.

Plants containing nuclear-located sequences encoding recombinases may be removed from a desired population by crossing, wherein the sequences may be lost due to segregation of this trait.

In other embodiments of the invention, the plant cell is one which already comprises an expressible construct which is integrated into the genome of the desired plastid(s), wherein the expressible construct comprises a nucleotide sequence encoding a recombinase. The expressible construct might, for example, have been introduced into the plastid genome by homologous recombination. In such cases, the recombinase must be under the control of an inducible promoter. Such an inducible promoter may have been introduced with the construct or the construct may have been integrated adjacent to an endogenous inducible promoter.

Thus in some embodiments of the invention, step (iii) comprises:
(iii) inducing the expression of a recombinase in the plant cell from an inducible promoter operably linked to a nucleotide sequence encoding a recombinase which is present in the plant cell, wherein the recombinase recognises the first and second site-specific recombination elements.

Preferably, the inducible promoter operably linked to a nucleotide sequence encoding a recombinase is present in the nuclear genome of the plant cell.

In other preferred embodiments, the inducible promoter operably linked to a nucleotide sequence encoding a recombinase is present in a plastid genome of the plant cell.

In yet other embodiments of the invention, the plant cell is transformed with a Recombinase Vector which comprises a promoter operably linked to a nucleotide sequence encoding a recombinase, either before step (i), simultaneously with step (i) or after step (i); or before step (ii), simultaneously with step (ii) or after step (ii).

The Recombinase Vector is a nucleic acid vector that comprises a promoter element that is capable of driving the expression of a downstream recombinase. The vector is preferably designed such that the recombinase is either expressed only or substantially only in plastids or is targeted specifically or substantially specifically to plastids.

The promoter in the Recombinase Vector must be one that is operable in the plant cell which is to be transformed. The promoter might, for example, be one derived from a plant or bacterial gene. Preferably, the promoter is plant-specific or plastid-specific. Most preferably, the promoter is an inducible promoter such as XVE (Zuo J, Niu QW, Chua NH. (2000) Technical advance: An estrogen receptor-based transactivator XVE mediates highly inducible gene expression in transgenic plants. Plant J. 24, 265-273.).

In the context of the present invention, the term "plant-specific" means plant-specific or substantially plant-specific. Similarly, the term "plastid-specific" means specific or substantially specific to plastids.

The promoter may or may not be an inducible promoter. If the Recombinase Vector is introduced to the plant cell before or during selection (step (ii)), it is preferable that the promoter is inducible. Examples of inducible promoters which are capable of operating in plants include light inducible promoters, metal inducible promoters, heat-shock promoters and other environmentally-inducible promoters.

Preferably, the promoter is an inducible promoter, for example an XVE promoter (Zuo J, Niu QW, Chua NH. (2000) Technical advance: An estrogen receptor-based transactivator XVE mediates highly inducible gene expression in transgenic plants. Plant J. 24, 265-273.) or a lac promoter.

In one embodiment of the invention, the Recombinase Vector comprises a promoter, operably linked to a nucleotide sequence encoding a plastid-targeting transit peptide and a recombinase.

Upon expression, a polypeptide product will be produced comprising a plastid-targeting transit peptide operably linked to a recombinase polypeptide. In this embodiment, the promoter may or may not be plastid-specific.

In the context of the present invention, the term "plastid-targeting transit peptide" means a peptide sequence which is capable of targeting the recombinase polypeptide to a plastid in a specific or substantially specific manner. Upon expression, the recombinase polypeptide will be produced and specifically imported into plastids by means of the plastid-targeting peptide.

Examples of plastid-targeting transit peptides include plastid-targeting transit peptides from plastid-targeted proteins.

Preferably, the plastid-targeting transit peptide is one which is capable of targeting the recombinase polypeptide to a chloroplast.

Most preferably, the plastid-targeting transit peptide is a plastid-targeting transit peptide from a stromal plastid targeted protein.

Specific examples of plastid-targeting transit peptides include: Transit peptide from AtABC1 (Simon Geir Møller, Tim Kunkel and Nam-Hai Chua. (2001) "A plastidic ABC protein involved in intercompartmental communication of light signaling", Genes and Dev. 15, 90-103.); from AtMinE1 (Jodi Maple, Nam-Hai Chua and Simon Geir Møller (2002) "The topological specificity factor AtMinE1 is required for correct plastid division site placement in Arabidopsis", Plant J. 31, 269-277); and from GIANT CHLOROPLAST 1 (Jodi Maple, Makoto T. Fujiwara, Nobutaka Kitahata, Tracey Lawson, Neil Baker, Shigeo Yoshida and Simon Geir Møller (2004) "GIANT CHLOROPLAST 1 is essential for correct plastid division in Arabidopsis". Current Biology. 14, 776-781)).

Most preferably, the Recombinase Vector comprises an XVE promoter, operably linked to a nucleotide sequence encoding a plastid-targeting transit peptide and CRE recombinase.

The Recombinase Vector may also comprise other elements, for example, the nptII gene (kanamycin resistance) to allow for selection of transformed cells.

Thus in some embodiments of the invention, step (iii) comprises:
(iii) transforming the plant cell with a Recombinase Vector comprising a promoter, a nucleotide sequence encoding a plastid-targeting transit peptide and a recombinase, wherein the recombinase is one which recognises the first and second site specific recombination elements.

Particularly preferably, step (iii) of the invention comprises:
(iii) transforming the plant cell with a Recombinase Vector comprising a promoter, a nucleotide sequence encoding a plastid-targeting transit peptide and a recombinase, wherein the promoter is capable of driving the expression of the nucleotide sequence encoding the plastid-targeting transit peptide and the recombinase in the plant cell, and wherein, upon expression in the plant cell, the recombinase polypeptide is targeted by the transit peptide to the plastid.

More preferably, the promoter is an inducible promoter.

The person skilled in the art will be aware of numerous methods for transforming plant cells with nucleic acid vectors. These include direct DNA uptake into protoplasts, PEG-mediated uptake to protoplasts, microparticle bombardment electroporation, micro-injection of DNA, micro-particle bombardment of tissue explants or cells, vacuum-infiltration of plant tissues, and T-DNA mediated transformation of plant tissues by *Agrobacterium.*

For transformation of the plant cell containing the selected plastid, any such suitable method may be used.

For targeting the genetic construct to plastids, biolistic transformation is preferred. This involves shooting nucleic acid vector-coated gold particles (micro-projectiles) into plastids of plant tissues, followed by selection of the transformed plastids and plant regeneration. Preferably, the plant tissue is a plant leaf, although callus, as for rice transformation, may also be used.

The method of the invention preferably also comprises the additional step of inducing the expression of the recombinase in the plant cell.

This step will take place after the Recombinase Vector/expressible construct and Transformation Cassette are both present in the plant cell. Preferably, this step will take place after selection of the plant cells on media lacking the plant-hormone cytokinin.

The expression of the recombinase may be induced by applying an inducing agent which results in the activation of the promoter which is present in the Recombinase Vector or endogenous promoter or expressible construct. The recombinase polypeptide is expressed and it then binds to the first and second site-specific recombination elements in the Excision Cassette, leading to the excision of that Cassette. (As will be understood by the person skilled in the art, one of the site-specific recombination elements and some adjacent sequence may be left in the plastid genome).

Once the nucleotide sequence encoding the plant-hormone biosynthetic polypeptide has been removed from the plastid genome, the promoter which is present in the Transformation Cassette will then be able to direct expression of the downstream transgene(s), thus producing the polypeptides(s) of interest.

In general, plants are regenerated in the presence of cytokinin (shoot formation) and auxin (root formation). In the case of regenerating plants containing the IPT gene (producing cytokinin), appropriate cells/tissues (e.g. leaf segments) will be placed on media containing auxin only (for root regeneration) whilst shoot regeneration will occur due to the presence of the IPT gene.

The invention also provides a method for making a transgene product, comprising the method for producing a transformed plant cell, as described hereinbefore, and additionally comprising purifying the transgene product from the plastids.

Yet a further particularly preferred embodiment provides a method for producing a transformed plant cell, the method comprising the steps:
(i) transforming the plant cell with a genetic construct,
   wherein the genetic construct comprises first and second homologous recombination elements flanking a Transformation Cassette, wherein the nucleotide sequences of the first and second homologous recombination elements are selected such that the Transformation Cassette is specifically targeted to one or more selected plastids and wherein the first and second homologous recombination elements direct the integration of the Transformation Cassette into the genome of the one or more selected plastids which are present in the plant cell,
   wherein the Transformation Cassette comprises:
   (a) a first promoter which is operable in said plant cell,
   (b) an Excision Cassette,
   (c) one or more transgenes,
   (d) a first terminator element,
   wherein the Excision Cassette comprises:
   (b1) a first site-specific recombination element,
   (b2) an optional second promoter
   (b3) a nucleotide sequence encoding a plant-hormone biosynthetic polypeptide, wherein the polypeptide is IPT,
   (b4) a second terminator element,
   (b5) a second site-specific recombination element,
   wherein the first and second site-specific recombination elements are capable of being recognised by a recombinase and wherein the first and second site-specific recombination elements are lox elements and the recombinase is Cre,
(ii) selecting for transformed plant cells on media which is lacking cytokinin;
   and (iii) expressing a Cre recombinase in the plant cell at a level which results in excision of the Excision Cassette from the plastid genome.

Also disclosed is a plant cell comprising a Transformation Cassette of the invention, a plant cell comprising a Recombinase Vector of the invention, and a plant cell comprising a Transformation Cassette and a Recombinase Vector of the invention.

Also disclosed is a transgenic plant comprising a Transformation Cassette of the invention, a transgenic plant comprising a Recombinase Vector of the invention, and a transgenic plant comprising a Transformation Cassette and a Recombinase Vector of the invention.

Also disclosed is a plant seed comprising a Transformation Cassette of the invention, a plant seed comprising a Recombinase Vector of the invention, and a plant seed comprising a Transformation Cassette and a Recombinase Vector of the invention.

Also disclosed is a plant plastid comprising a Transformation Cassette of the invention, a plant plastid comprising a Recombinase Vector of the invention, and a plant plastid comprising a Transformation Cassette and a Recombinase Vector of the invention.

Additionally, the invention provides a plant cell obtainable or obtained using a method of the invention.

### BRIEF DESCRIPTION OF THE FIGURES

### Figure 1

### Schematic diagram showing the overall principle of the IPT gene excision and YFP gene activation.

pPTI001-YFP containing the IPT gene sandwiched between two lox sites which allows for CRE-mediated IPT excision after regeneration. The removal of the IPT gene results in simultaneous transgene (YFP) activation.

### Figure 2

### CRE induced excision of the IPT cassette in pPTI001-YFP.

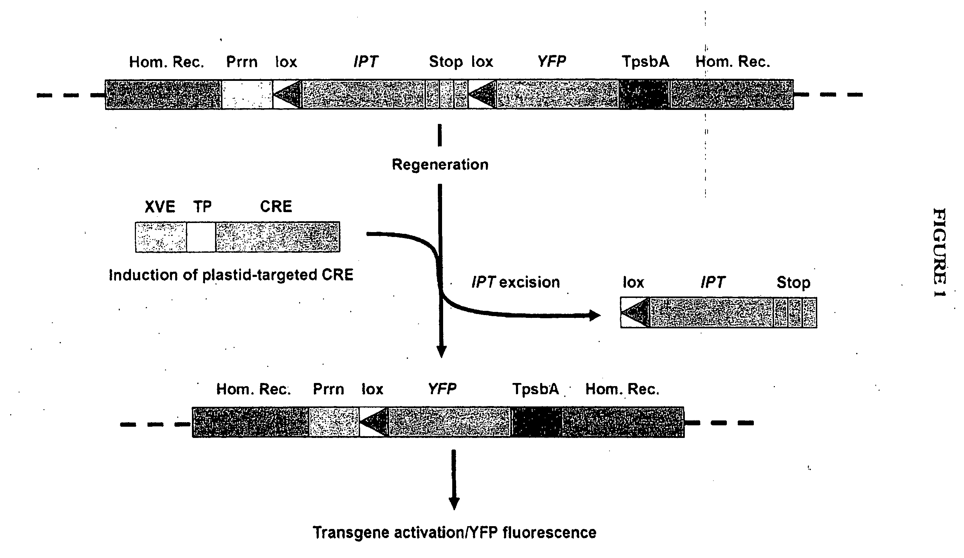

Bright field (A) and fluorescence (B) images of *E. coli* DH5α cells containing the pPTI001-YFP vector. Bright field (C) and fluorescence (D) images of *E. coli* DH5α cells containing both the pPTI001-YFP and pER10-TP.CRE vectors (Figure 1). CRE induced excision of the IPT cassette in pPTI001-YFP results in constitutive expression of YFP from the Prrn promoter. (E) PCR confirmation of CRE induced excision of the IPT cassettes in the pPTI001-YFP vector using primers spanning the TrbcL and TpsbA terminators (Figure 3). M: molecular weight marker.

### Figure 3

### Schematic diagram of the (A) pPTI001 and (B) pPTI001-YFP vectors.

**HOM1:** Homologous recombination sequence (nt 104091-105380, accession Z00044 *Nicotiana tabacum* chloroplast genome DNA); Prrn: Plastidic ribosomal RNA (*rrn*) operon promoter (nt 59034-59303, accession Z00044 *Nicotiana tabacum* chloroplast genome DNA); IPT: isopentenyltranferase gene from *Agrobacterium tumefaciens;* TrbcL: *Ribulose-1,5-Bisphosphate Carboxylase*/*Oxygenase polyA* addition sequence (nt 102539-102685, accession Z00044 *Nicotiana tabacum* chloroplast genome DNA); YFP: Yellow fluorescence protein; TpsbA: *psbA* polyA addition sequence; HOM2: Homologous recombination sequence (nt 105381-106370, accession Z00044 *Nicotiana tabacum* chloroplast genome DNA).

### Figure 4

### Schematic diagram of the modified pPTI001-YFP vector containing protein purification tags (pPTI001 vector series).

(A) pPTI001a-YFP, pPTI001-YFP containing an N-terminal influenza hemagglutinin-HA-epitope tag (HA3).
(B) pPTI001b-YFP, pPTI001-YFP containing six N-terminal histidine amino acids (HIS6).
(C) pPTI001c-YFP, pPTI001-YFP containing a C-terminal influenza hemagglutinin-HA-epitope tag (HA3).
(D) pPTI001d-YFP, pPTI001-YFP containing six C-terminal histidine amino acids (HIS6).

To allow for the use of EcoRV as a cloning site downstream from YFP in (C) pPTI001c-YFP and (D) pPTI001d-YFP the IPT DNA sequence was modified changing adenine to guanidine at nucleotide position 519 (+1 taken as adenine in the start codon) thereby removing an endogenous EcoRV site.

### Figure 5

### Schematic diagram of the (A) pPTI002 and (B) pPTI002-YFP vectors.

**HOM1:** Homologous recombination sequence (nt 104091-105380, accession Z00044 *Nicotiana tabacum* chloroplast genome DNA); Prrn: Plastidic ribosomal RNA (*rrn*) operon promoter (nt 59034-59303, accession Z00044 *Nicotiana tabacum* chloroplast genome DNA); IPT: isopentenyltranferase gene from *Agrobacterium tumefaciens;* **TrbcL:** *Ribulose-1,5-Bisphosphate Carboxylase*/*Oxygenase polyA* addition sequence (nt 102539-102685, accession Z00044 *Nicotiana tabacum* chloroplast genome DNA); **YFP:** Yellow fluorescence protein; **TpsbA:** *psbA* polyA addition sequence; **HOM2:** Homologous recombination sequence (nt 105381-106370, accession Z00044 *Nicotiana tabacum* chloroplast genome DNA).

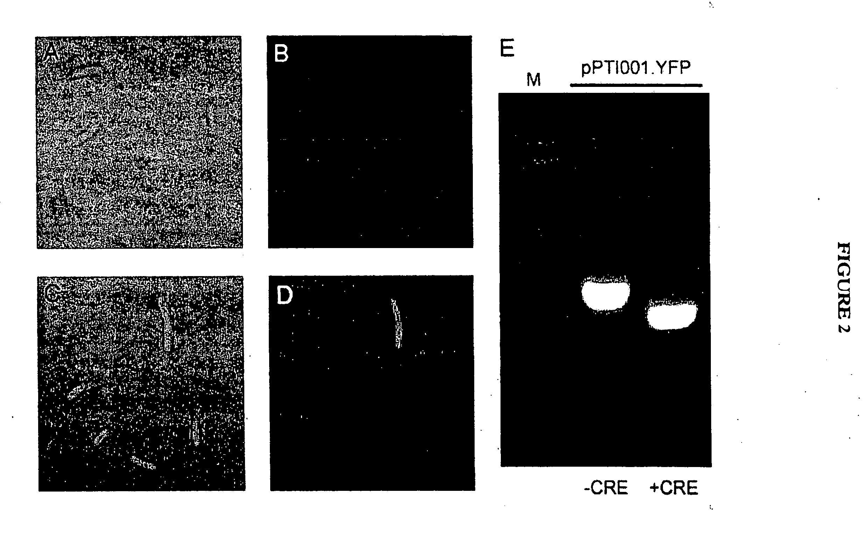

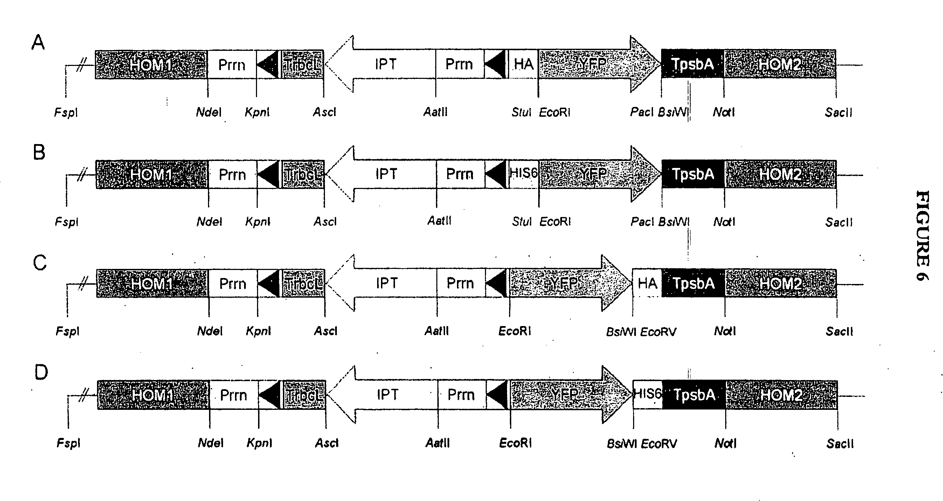

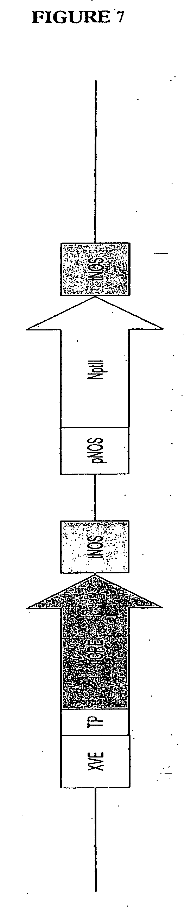

The construction of the vector series pPTI002 (see also Figure 6) is to ensure that there is no leaky expression from the Prrn promoter to the transgene in question (in this case YFP) prior to CRE-mediated excision. Although this does not seem to present a real problem, as shown in Figure 2, we have placed the IPT gene and the YFP gene in opposite orientations.

### Figure 6

### Schematic diagram of the modified pPTI002-YFP vector containing protein purification tags (pPTI002 vector series).

(A) pPTI002a-YFP, pPTI002-YFP containing an N-terminal influenza hemagglutinin-HA-epitope tag (HA).
(B) pPTI002b-YFP, pPTI002-YFP containing six N-terminal histidine amino acids (HIS6).
(C) pPTI002c-YFP, pPTI002-YFP containing a C-terminal influenza hemagglutinin-HA-epitope tag (HA).
(D) pPTI002d-YFP, pPTI002-YFP containing six C-terminal histidine amino acids (HIS6).

To allow for the use of EcoRV as a cloning site downstream from YFP in (C) pPTI002c-YFP and (D) pPTI002d-YFP, the IPT DNA sequence was modified changing adenine to guanidine at nucleotide position 519 (+1 taken as adenine in the start codon) thereby removing an endogenous EcoRV site.

### Figure 7

### Schematic diagram of pER10/TPCRE.

XVE acts as the inducible promoter that drives TP-CRE (Transit peptide fused to CRE) expression. Selection of this transgene is by Kanamycin resistance conferred by the *nptII* gene.

### Figure 8

### Selection and regeneration of positive transplastomic plants using the IPT selectable marker.

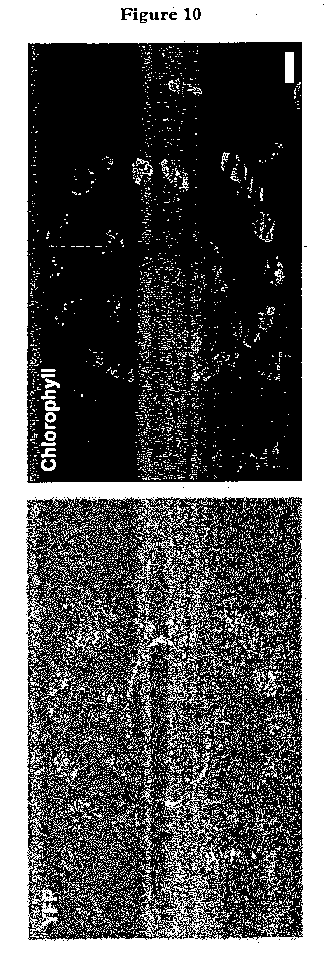

A) First round of selection after plastid transformation showing the presence of new shoots in the absence of cytokinin.
B) Second round of selection from the primary transformant shown in A.
C) Regenerated transplastomic tobacco plant used for CRE infiltration and GFP activation (Figure 10).

### Figure 9

### Confirmation of transgene insertion into the tobacco plastid genome.

Insertion of pPTI001/YFP into the tobacco chloroplast genome at the homologous recombination sites was confirmed using a primer in the flanking region of the tobacco chloroplast genome and a primer that anneals to TrbcL terminator within the cassette.
Lanes shown: WT, wild-type; #1, regenerant 1; #2 regenerant 2; M, marker.

### Figure 10

### Induction of YFP expression in tobacco leaf cells.

Regenerants transplastomic tobacco plant harbouring the pPTI001/YFP transformation cassette analysed by fluorescence microscopy after infiltration and induction of pER10/TP.CRE. Extended focus images of reconstituted YFP fluorophore (YFP) and chlorophyll autofluorescence (Chlorophyll) were captured by epifluorescence microscopy using Volocity II software. Scale bar = 5 µm.

### Figure 11

### Plastid transformation vector pPTI005:

**HOM1:** Homologous recombination sequence (nt 104091-105380, accession Z00044 *Nicotiana tabacum* chloroplast genome DNA); **PpsbA:** Plastidic psbA promoter; **aadA:** spectinomycin adenyltransferase gene; **T7:** T7 transcription terminator sequence: **Prrn:** Plastidic ribosomal RNA (*rrn*) operon promoter (nt 59034-59303, accession Z00044 *Nicotiana tabacum* chloroplast genome DNA); **IPT:** isopentenyltranferase gene from *Agrobacterium tumefaciens;* **TrbcL:** *Ribulose-1,5-Bisphosphate Carboxylase*/*Oxygenase polyA* addition sequence (nt 102539-102685, accession Z00044 *Nicotiana tabacum* chloroplast genome DNA); **TpsbA:** *psbA* polyA addition sequence; **HOM2:** Homologous recombination sequence (nt 105381-106370, accession Z00044 *Nicotiana tabacum* chloroplast genome DNA).

### Figure 12

### Plastid transformation vector pPTI007:

**HOM3:** Homologous recombination sequence (nt 102925-101857, accession Z00044 *Nicotiana tabacum* chloroplast genome DNA); **PpsbA:** Plastidic psbA promoter; **aadA:** spectinomycin adenyltransferase gene; T7: T7 transcription terminator sequence: **Prrn:** Plastidic ribosomal RNA (*rrn*) operon promoter (nt 59034-59303, accession Z00044 *Nicotiana tabacum* chloroplast genome DNA); **IPT:** isopentenyltranferase gene from *Agrobacterium tumefaciens;* **TrbcL:** *Ribulose-1,5-Bisphosphate Carboxylase*/*Oxygenase polyA* addition sequence (nt 102539-102685, accession Z00044 *Nicotiana tabacum* chloroplast genome DNA); **TpsbA:** *psbA* polyA addition sequence; HOM4: Homologous recombination sequence (nt 100933-100130, accession Z00044 *Nicotiana tabacum* chloroplast genome DNA).

### Figure 13

### Plastid transformation vector pPTI008:

**HOM3:** Homologous recombination sequence (nt 102925-101857, accession Z00044 *Nicotiana tabacum* chloroplast genome DNA); Prrn: Plastidic ribosomal RNA *(rrn)* operon promoter (nt 59034-59303, accession Z00044 *Nicotiana tabacum* chloroplast genome DNA); IPT: isopentenyltranferase gene from *Agrobacterium tumefaciens;* **aadA:** spectinomycin adenyltransferase gene; **TrbcL:** *Ribulose-1,5-Bisphosphate Carboxylase*/*Oxygenase polyA* addition sequence (nt 102539-102685, accession Z00044 *Nicotiana tabacum* chloroplast genome DNA); **TpsbA:** *psbA* polyA addition sequence; HOM4: Homologous recombination sequence (nt 100933-100130, accession Z00044 *Nicotiana tabacum* chloroplast genome DNA). The *IPT.aadA* cassette is constructed as an operon. Both of the *IPT* and *aadA* genes are preceded by a shine delgardo sequence and a six base pair spacer.

### Figure 14

### Plastid transformation vector pPTI009:

**HOM3:** Homologous recombination sequence (nt 102925-101857, accession Z00044 *Nicotiana tabacum* chloroplast genome DNA); **Prrn:** Plastidic ribosomal RNA (*rrn*) operon promoter (nt 59034-59303, accession Z00044 *Nicotiana tabacum* chloroplast genome DNA); **IPT:** isopentenyltranferase gene from *Agrobacterium tumefaciens;* **aadA:** spectinomycin adenyltransferase gene; **TrbcL:** *Ribulose-1,5-Bisphosphate Carboxylase*/*Ozygenase polyA* addition sequence (nt 102539-102685, accession Z00044 *Nicotiana tabacum* chloroplast genome DNA); **TpsbA:** *psbA* polyA addition sequence; **HOM4:** Homologous recombination sequence (nt 100933-100130, accession Z00044 *Nicotiana tabacum* chloroplast genome DNA). The *IPT.aadA* cassette is expressed as a fusion protein. The *IPT* a gene is preceded by a shine delgardo sequence and a six base pair spacer and the two open reading frames are separated by an eight glycine linker sequence.

### Figure 15

### Plastid transformation vectors pinPTI.

Each pPTI vector contains a constitutively expressed *LacI* gene. pPTI003 is shown as an example. Modified Prrn promotors (PrrnL) will be inserted upstream of the IPT.aadA cassette, allowing inducible, controlled expression of the IPT.aadA cassette. (A) pind1PTI, pPTI containing the LacI open reading frame under the control of the Prrn promoter sequence and modified PrrnL promoter upstream of the IPT.aadA cassette. (B) pind2PTI, pPTI containing the *LacI* open reading frame under the control of the T7 promoter sequence and modified PrrnL promoter upstream of the IPT.aadA cassette. **HOM1:** Homologous recombination sequence (nt 104091-105380, accession Z00044 *Nicotiana tabacum* chloroplast genome DNA); **Prrn:** Plastidic ribosomal RNA (*rrn*) operon promoter (nt 59034-59303, accession Z00044 *Nicotiana tabacum* chloroplast genome DNA); **IPT:** isopentenyltranferase gene from *Agrobacterium tumefaciens;* **aadA:** spectinomycin adenyltransferase gene; **TrbcL:** *Ribulose-1,5-Bisphosphate CarboxylaselOxygenase polyA* addition sequence (nt 102539-102685, accession Z00044 *Nicotiana tabacum* chloroplast genome DNA); **TpsbA:** *psbA* polyA addition sequence; HOM2: Homologous recombination sequence (nt 105381-106370, accession Z00044 *Nicotiana tabacum* chloroplast genome DNA). The *IPT.aadA* cassette is constructed as an operon. Both of the *IPT* and *aadA* genes are preceded by a shine delgardo sequence and a six base pair spacer.

### Figure 16

### Plastid transformation vectors pPTI+

Each pPTI vector will be modified to contain a modified Prrn-trbcL (Prrn promoter construct, composed of the Prrn promter and *rbcL* 5' translation control region, to ultimately produce higher levels of expression of the foreign proteins. pPTI003 is shown as an example. **HOM1:** Homologous recombination sequence (nt 104091-105380, accession Z00044 *Nicotiana tabacum* chloroplast genome DNA); **Prrn-t:** Plastidic ribosomal RNA (*rrn*) operon promoter (nt 59034-59303, accession Z00044 *Nicotiana tabacum* chloroplast genome DNA) and *rbcL* 5' translation control region; **IPT:** isopentenyltranferase gene from *Agrobacterium tumefaciens;* **aadA:** spectinomycin adenyltransferase gene; **TrbcL:** *Ribulose-1,5-Bisphosphate CarboxylaselOxygenase polyA* addition sequence (rit 102539-102685, accession Z00044 *Nicotiana tabacum* chloroplast genome DNA); **TpsbA:** *psbA* polyA addition sequence; **HOM2:** Homologous recombination sequence (nt 105381-106370, accession Z00044 *Nicotiana tabacum* chloroplast genome DNA). The *IPT.aadA* cassette is constructed as an operon. Both of the *IPT* and *aadA* genes are preceded by a shine delgardo sequence and a six base pair spacer.

### EXAMPLES

### Example 1

The plastid transformation vectors pPTI001 and pPTI002 were constructed as detailed in Figure 3 and Figure 5 using a 1289 bp homologous recombination sequence (104091-105380 nt) and a 989 bp homologous recombination sequence (105381-106370 nt) from the chloroplast genome from *Nicotiana tabacum* (Shinozaki K, Ohme M, Tanaka M, Wakasugi T, Hayashida N, Matsubayashi T, Zaita N, Chunwongse J, Obokata J, Yamaguchi-Shinozaki K, Ohto C, Torazawa K, Meng BY, Sugita M, Deno H, Kamogashira T, Yamada K, Kusuda J, Takaiwa F, Kato A, Tohdoh N, Shimada H, Sugiura M. (1986) The complete nucleotide sequence of the tobacco chloroplast genome: its gene organization and expression. EMBO J. 5, 2043-2049.) on either side of the gene cassette. Shorter homologous recombination sequences from the *Nicotiana tabacum* chloroplast DNA (157 and 143 bp in length) have also been used showing a 3-fold increase in plastid transformation efficiencies.

For proof-of-principle purposes, the Yellow Fluorescent Protein (YFP) reporter gene was cloned into pPTI0001 and pPTI002 to generate pPTI001-YFP (Figure 3) and pPTI002-YFP (Figure 5). These vectors where then bombarded into tobacco leaves as detailed in Appendix 1. For transformation experiments, leaves from plants growing in the greenhouse were used instead of in Magenta Box as this increases the transformation efficiency. Leaves were sterilized in 10% Super Bleach Sterilizer (Coventry) for ten minutes and washed in sterilized water three times. The leaves were then cut to fit the plates for bombardment. Following these strategies, the transformation efficiency increased nearly ten times.

Because the pPTI001 and pPTI002 vector series also functions in bacteria, we tested whether the CRE-mediated excision of the IPT gene was functional ultimately leading to YFP gene activation and YFP fluorescence in *E. coli* cells. A schematic diagram showing the principal of the system is shown in Figure 1. Chemically competent *E. coli* DH5α cells were produced and transformed with the vector pER10-TP.CRE, which constitutively expresses the TP.CRE fusion protein, and selected on LB media containing spectinomycin. Subsequently chemically competent E. coli DH5α cells containing the pER10-TP.CRE vector were produced and transformed with pPTI001-YFP. Cells containing both vectors were selected for on LB media containing spectinomycin and chloramphenicol. Single colonies were inoculated into LB media containing spectinomycin and chloramphenicol and grown to an OD600 of 0.4 before analysis for YFP expression on a Nikon TE-2000U inverted fluorescence microscope equipped with filters for YFP (exciter HQ500/20, emitter S535/30) fluorescence and a Hamamatsu Orca ER 1394 cooled CCD camera. Images were captured using Openlab software (Improvision). PCR verification of IPT excision was carried out on plasmid DNA prepared from the *E. coli* cells and the primers TrbcL-F and TpsbA-R which span the IPT cassette region. Figure 2 shows no excision of the IPT gene prior to addition of CRE (Figures 2A and 2E) whilst after CRE addition the IPT gene is removed at the molecular level (Figure 2E) leading to YFP expression (Figure 2D).

### Example 2

### Expression of proteins with toxic effects on plant development

The expression of high levels of foreign protein in plants can lead to detrimental effects on plant development because of toxic effects. The described system overcomes this by combining insertion of the transgene(s) into the plastid genome where it remains dormant until the IPT selectable marker gene is removed by CRE/lox mediated recombination.

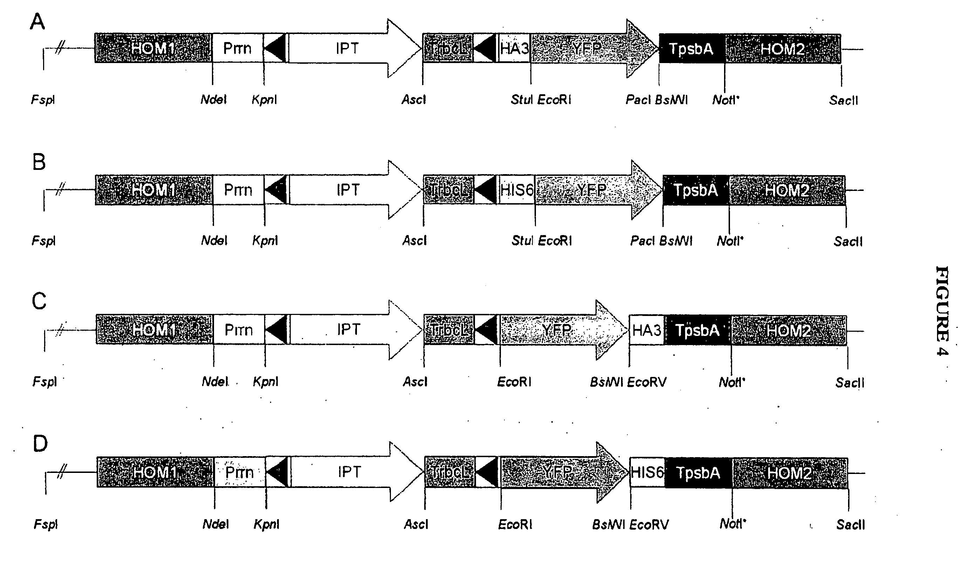

The transgene encoding the "plant-toxic" protein is inserted into one of the pPTI001 or pPTI002 vectors (Figure 3 and 5) between the TrbcL and the TspbA polyA addition sequences in the pPTI001 vector series or between the Prrn promoter and the TspbA polyA addition sequence in the pPTI002 vector series and the construct transformed into plastids using the protocol shown in Appendix 1 followed by cytokinin-mediated selection and regeneration. Once regenerated, the IPT gene is removed by CRE-mediated recombination and the toxic transgene is activated leading to minimal adverse effects on initial plant regeneration. Once expressed, the recombinant protein can be purified using one of the affinity tags present in either the pPTI001 or pPTI002 vector series shown in Figures 4 and 6.

### Example 3

### Expression of proteins that have a role in plastids

Plastids play an integral role during plant development and it may therefore be of interest to express proteins (plant or non-plant) inside plastids that would have a positive effect on plant growth, development and/or confer modified characteristics to the plant as whole. Due to the high expression levels of transgenes in plastids coupled to the fact that the IPT as a selectable marker gene does not lead to the generation of spontaneous ribosomal mutants (as with the common spectinomycin selectable marker gene), the present invention represents an ideal system for this application.

The transgene is inserted into one of the pPTI001 or pPTI002 vectors (Figure 3 and 5) between the TrbcL and the TspbA polyA addition sequences in the pPTI001 vector series or between the Prrn promoter and the TspbA polyA addition sequence in the pPTI002 vector series and the construct transformed into plastids using the protocol shown in Appendix 1 followed by cytokinin-mediated selection and regeneration. Once regenerated, the IPT gene is removed by CRE-mediated recombination and the transgene is activated leading to minimal adverse effects on initial plant regeneration.

### Example 4

### High level expression of eukaryotic proteins

There are often problems associated with expression of eukaryotic proteins in bacterial systems due to insolubility and/or lack of post-translational modifications. Similarly, the expression of eukaryotic proteins in mammalian cells is expensive and labour intensive. The present system can be used for the expression of eukaryotic proteins in plastids using IPT marker gene selection and transgene activation.

As described in Examples 2 and 3, any gene encoding a eukaryotic protein may be inserted into one or all of the pPTI001 series or the pPTI002 series of vectors followed by transformation, selection and regeneration as described previously. Following regeneration, the expressed protein may be purified using the affinity tags shown in Figures 4 and 6 and used for downstream applications.

A non-exclusive list of possible eukaryotic protein families that will be expressed includes antibodies, enzymes, enzyme inhibitors and design peptides.

### Example 5

### High level expression of prokaryotic proteins

Due to the endosymbiotic origin of plastids, it is possible to express prokaryotic proteins in plastids. As described in Examples 2 and 3, any gene encoding a prokaryotic protein may be inserted into one or all of the pPTI001 series or the pPTI002 series of vectors followed by transformation, selection and regeneration as described previously. Following regeneration, the expressed protein may be purified using the affinity tags shown in Figures 4 and 6 and used for downstream applications.

### Example 6

### Coordinated expression of multiple proteins from the same promoter

Due to the endosymbiotic origin of plastids, it is possible to express operon-like gene structures in plastids. This entails the coordinated and simultaneous expression of multiple proteins under the regulation of a single promoter. As described in Examples 2 and 3, multiple genes encoding protein of both prokaryotic and eukaryotic origin (or a mix thereof) may be inserted into one or all of the pPTI001 series or the pPTI002 series of vectors followed by transformation, selection and regeneration as described previously.

### Example 7

### Expression of YFP from pPTI001/YFP in plastids of tobacco:

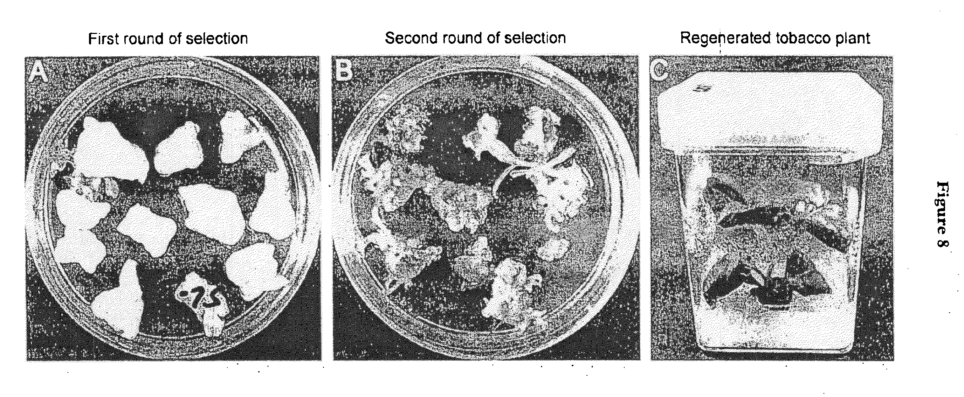

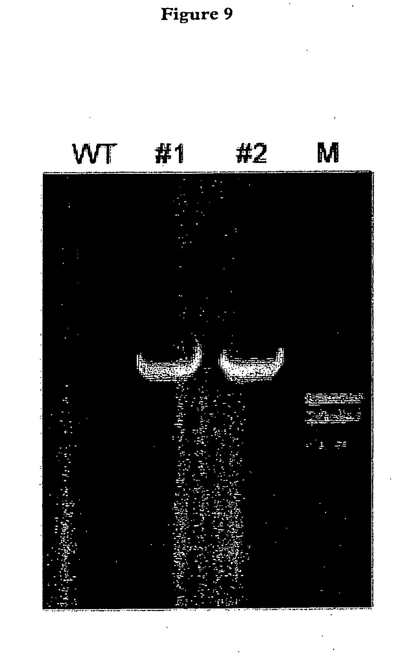

pPTI001/YFP vector was bombarded into tobacco leaf cells and regenerants selected on media containing only auxin and on media lacking all hormones. Regenerants were obtained (Figure 8) and transferred to secondary selection media containing auxin to induce root formation before transfer to soil. The incorporation of the transformation cassette in the tobacco plastid genome was confirmed by PCR using vector-specific primers and primers in the flanking region of the tobacco chloroplast genome (Figure 9). Leaves from the regenerated plants were infiltrated with pER10/TP.CRE, a binary vector expressing the TP.CRE fusion, followed by induction. After 72 hours the infiltrated tissue was analysed by fluorescence microscopy revealing cells containing GFP fluorescing chloroplasts (Figure 10).

### Example 8

### Additional and new plastid transformation vectors

To optimize the selection and regeneration system further a new series of plastid transformation vectors have been constructed. The following modifications and additions have been made:
1) Inclusion of the aadA gene in addition to the IPT gene for double selection purposes.
2) Inclusion of new homologous recombination sites (HOM3 and HOM4) that may increase more efficient homologous recombination and transgene insertion.
3) Inclusion of an IPTG inducible promoter.
4) Inclusion of a modified Prrn promoter for higher expression levels.

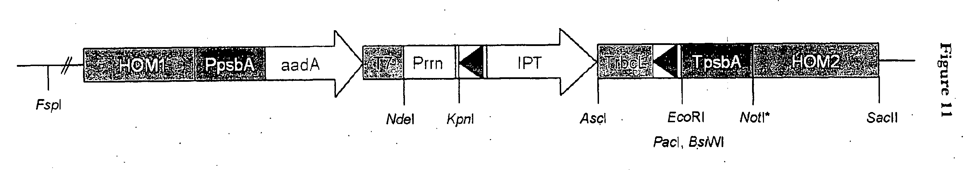

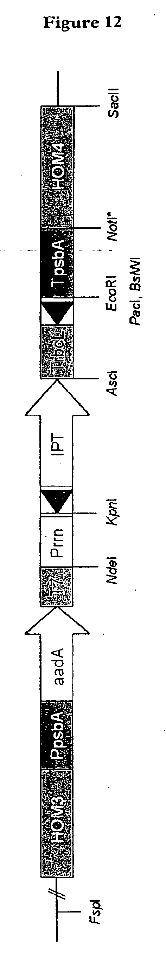

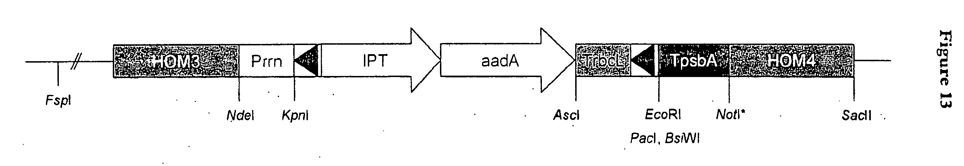

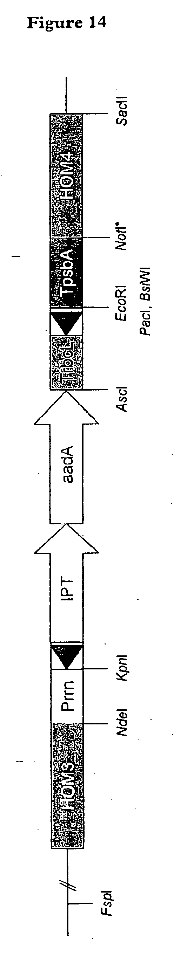

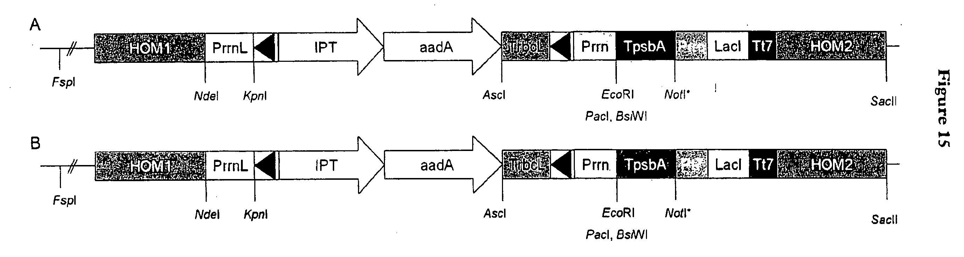

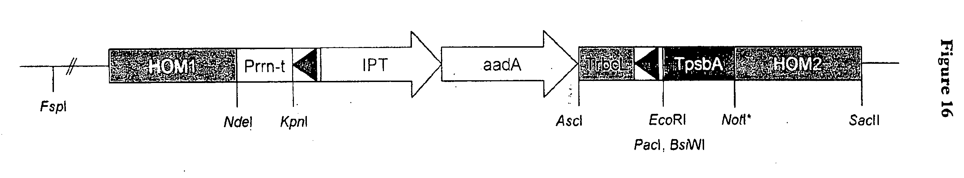

The details of the vectors are shown in Figures 11-16. All the exemplified vectors contain the IPT selectable marker gene.

### Appendix 1

### PROTOCOL FOR CHLOROPLAST TRANSFORMATION

### TIME COURSE

The standard procedures produce transformed plants in 3-5 months.
- Bombardment to first shoot on: 3-8 weeks
- Subculture production of homoplastic plants: 3-4 weeks
- Growth in soil prior to analysis: 1-2 weeks

### EQUIPMENT SET UP

### Helium Gun Biorad PDS 1000

- Rupture disk PSI: 1100
- Gap between rupture disk retaining cap and macrocarrier over cover lid: ¼"
- Spacer rings below stopping screen support: 2
- Level of macrocarrier launch assembly: 1 (from top)
- Level of Petri dish holder: 4 (from top)
- Vacuum inflow rate: Maximum
- Vacuum release rate: Attenuate the release so it approximates the speed of vacuum inflow.

### STOCK SOLUTIONS

- 2.5 M CaCl₂ autoclave or filter sterilize
- 1 M Spermidine Free Base in sterilize H²O
- dH₂O
- DNA at 1 µg/µl in dH₂O or 1X TE
- 100% Ethanol
- 70% Ethanol

### CONSUMABLES

- 1100 psi rupture disks
- Stopping screens
- Macrocarriers
- Gold particles

### PREPARATION OF THE DNA-GOLD PARTICLE MIX

- 50 mg of Gold particles are suspended in 1 ml of absolute ethanol as stock.
- Take 0.25 ml of Gold stock suspension and microfuge for 5 seconds. Remove Ethanol and wash 3 times with sterile distilled H₂O, microfuging 3 minutes between washings.
- Resuspend Gold in 0.25 ml dH₂O.
- Aliquot 50 µl of Gold-H₂O suspension into Eppendorf tubes.
- Into each Eppendorf add the following in succession:
   10 µl DNA at 1 µg/µl
   50 µl of 2.5 M CaCl₂
   20 µl of 0.1 M Spermidine free base
- Vortex for 5 minutes at highest speed.
- Add 200 µl of absolute ethanol to each tube.
- Spin in microfuge at 3000 rpm for 2 seconds.
- Remove supernatant as much as possible and rinse pellet in absolute ethanol once, microfuging at 3000 rpm for 2 seconds.
- Resuspend pellet in 30 µl absolute ethanol (makes 4-5 shots). Store mixture on ice.

### PREPARING THE BIOLISTIC GUN AND CONSUMABLES

- Sterilize the gun vacuum chamber and surfaces with 70% ethanol or 70% isopropanol.
- Sterilize the rupture disks and macrocarrier holders in 70% ethanol for 10 minutes. Air dry in hood.
- Soak the macrocarriers in absolute ethanol to remove all traces of H₂O. Air dry in hood.
- Open the helium tank. Set the helium tank regulator to 1300 psi (or 200 psi over the rating of the rupture disks).

### BOMBARDMENT

- Snap the macrocarriers into their holders.
- Pipet 5 µl of the gold-DNA mixture onto the centre of the macrocarrier. The mixture should spread out evenly with few chunks.
- Place a rupture disk in the holder ring and tighten ring to the helium barrel.
- Place a stopping screen and macrocarrier with gold-DNA (in holder) into the retaining assembly and screw down.
- Place assembly into the vacuum chamber at level 1 (first from top).
- Place sample at level 4. Remove Petri dish lid.
- Turn on vacuum pump.
- Pull a vacuum to 27-29 in.Hg.
- Press and hold fire button till the rupture disk breaks.
- Release vacuum and remove sample.
- Remove rupture disk, macrocarrier and stopping screen.
- Repeat if desired.
- At the end of the experiment, turn off the helium tank. Pull a vacuum in the gun and release the remaining helium through the gun, then turn off the helium regulator.

The key to successful bombardment is usually in the spread of particles on the macrocarrier. The ethanol/gold/DNA mixture should quickly spread out over the centre of the macrocarrier. The resulting spread should be a very fine dusting of particles, evenly spread and containing few chunks. Chunk causes increased cell death.

Each 30 µl Gold-DNA mix gives four or five bombardments. The remaining mixture is usually too dense for good results.

### TOBACCO PREPARATION AND REGENERATION MEDIA

| | | |
|---|---|---|
| MS: | MS salts and vitamins (1X) | |
| | 30 g/l sucrose | |
| | 6 g/l phytagar | |
| | pH 5.8 | Autoclave |
| | | |
| RMOP: | MS salts (1X) | |
| | 1 mg/l BAP | |
| | 0.1 mg/l NAA | |
| | 1 mg/l Thiamine | |
| | 100 mg/l inositol | |
| | 30 g/l sucrose | |
| | 6 g/l phytagar | |
| | pH 5.8 | Autoclave |
| | | |
| RMOP- BAP | | |
| | pH 5.8 | Autoclave |
| | | |
| MS + | MS media | |
| | 1 mg/l IBA (indole-3-butyric acid) | |
| | 2 µM 17-β-estradiol | |
| | pH 5.8 | Autoclave |

### TISSUE CULTURE

- Tobacco plants are micropropagated using sterile technique in magenta boxes containing MS media.
- Expanded leaves are excised and placed abaxial surface up on a Whatman filter paper laying on top of RMOP media. The leaves are allowed to desiccate slightly (1-2 hours) prior to bombardment on the filter paper. Each bombardment can treat 1-3 leaves covering approx. 1/3 of the 9 cm Petri dish.
- Post bombardment, the plates are sealed and the leaves are left at 12:12 photoperiod at 24°C for 2 days.
- After two days the leaves are cut into sections approx. 5 mm square and placed abaxial side down on RMOP media lacking BAP.
- Green shoots can be collected from the bleached explants in 3-8 weeks.
- Leaves from these shoots are cut up (2mm square) and subcultured in the same selective media for approx. 4 weeks.
- Typically 4 shoots are collected per initial subcultured shoots. These are rooted in tubes containing MS media + 1mg/l IBA and 2 µM 17-β-estradiol.
- Rooted shoots are transferred to soil approx. 3-5 weeks after isolation. Plants are allowed to grow in standard tobacco conditions (16:8 photoperiod at 25°C).

### NOTES

1. Tobacco leaves do not have to lay completely flat for bombardment.
2. Tobacco leaves will lose their turgor after 2 days on filter paper. This is OK.
3. Do minimum amount of transferring. The maximum amount of transferring that needs to be done:
   a. Excise leaves from plants grown in MS. Place leaves on filter paper on RMOP.
   b. 2 days post bombardment place leaf explants on RMOP - BAP
   c. 3-8 weeks later, remove leaves from green shoots, cut, then transfer to RMOP-BAP.
   d. Collect 4 shoots, transfer to MS+ 1mg/l IBA+ 2 µM 17-β-estradiol in individual tubes.
4. Transformation frequency for an average experiment is anywhere from 1.5 stably transformed plants to 0.3 stably transformed plants per bombardment.

### MEDIA FOR TOBACCO CHLOROPLAST TRANSFORMATION

| **Name** | **Compositions** | **Container** | **Time⁴** |
|---|---|---|---|
| MSS: | MS Salts and Vitamins (1X) | Magenta Box | |
| | 30 g/l Sucrose | | |
| | 6 g/l Phytagar | | |
| | pH 5.8 | | |
| | Autoclave | | |
| | | | |
| MFB¹ | MS Salts (1X) | Petri Dish (9 cm) | 2 days |
| | 1 mg/l BAP | | |
| | 0.1 mg/l NAA | | |
| | 1 mg/l Thiamine | | |
| | 100 mg/l Inositol | | |
| | 30 g/l Sucrose | | |
| | 6 g/l Phytagar | | |
| | pH 5.8 | | |
| | Autoclave | | |
| | | | |
| MTS² | MFB minus BAP | Deep Petri Dish | 3-10 weeks⁵ |
| | | | |
| MFR³ | MSS | Magenta Box | 3-5 weeks |
| | 1 mg/l IBA (indole-3-butyric acid) | | |
| | 2 µM 17-β-estradiol | | |

| | | | |
|---|---|---|---|
| Notes: 1. MFB: media for bombardement 2. MTS: media for transgenic selection 3. MFR: media for rooting 4. Time: time that tobacco leaves stay in the media 5. This time includes second selection time | | | |

## Claims

1. A method for producing a transformed plant cell, the method comprising the step:
(i) transforming the plant cell with a genetic construct,
wherein the genetic construct comprises first and second homologous recombination elements flanking a Transformation Cassette, wherein the nucleotide sequences of the first and second homologous recombination elements are selected such that the Transformation Cassette is specifically targeted to one or more selected plastids and wherein the first and second homologous recombination elements direct the integration of the Transformation Cassette into the genome of the one or more selected plastids which are present in the plant cell,
wherein the Transformation Cassette comprises:
(a) a first promoter which is operable in said plant cell,
(b) an Excision Cassette,
(c) one or more transgenes,
(d) a first terminator element,
wherein the Excision Cassette comprises:
(b1) a first site-specific recombination element,
(b2) an optional second promoter
(b3) a nucleotide sequence encoding a plant-hormone biosynthetic polypeptide, wherein the plant-hormone biosynthetic polypeptide is IPT (isopentenyl transferase),
(b4) a second terminator element,
(b5) a second site-specifc recombination element,
wherein the first and second site-specific recombination elements are capable of being recognised by a recombinase, wherein the method additionally comprises the step:
(ii) selecting for transformed plant cells on media which is lacking cytokinin.

2. A method as claimed in claim 1, wherein the method additionally comprises the step:
(iii) expressing a recombinase in the plant cell, wherein the recombinase is one which recognises the first and second site-specific recombination elements, and optionally regenerating a plant from the transformed plant cell by adding (A) cytokinin and auxin or (B) auxin.

3. A method as claimed in any one of the preceding claims, wherein:
(A) the first and second site-specific recombination elements are lox sites; and/or
(B) the recombinase is a Cre recombinase; and/or
(C) at least one of the one or more transgenes codes for an antibody, antibiotic, herbicide, vaccine antigen, enzyme, enzyme inhibitor or design peptide; and/or
(D) the nucleotide sequences of the homologous recombination elements are selected such that no or essentially no Transformation Cassettes become integrated into the nuclear genome of the plant.

4. A method as claimed in claim 2 or claim 3, wherein
(A) step (iii) comprises inducing the expression of a recombinase in the plant cell from an inducible promoter operably linked to a nucleotide sequence encoding a recombinase which is present in the plant cell, wherein the recombinase recognises the first and second site-specific recombination elements; or
(B) step (iii) comprises transforming the plant cell with a Recombinase Vector which comprises a promoter operably linked to a nucleotide sequence encoding a recombinase, either before step (i), simultaneously with step (i) or after step (i) or before step (ii), simultaneously with step (ii) or after step (ii), preferably wherein the Recombinase Vector comprises a promoter operably linked to a nucleotide sequence encoding a plastid-targeting transit peptide and a recombinase, more preferably wherein the plastid-targeting transit peptide is one which is capable of targeting the recombinase to a chloroplast, optionally wherein the promoter is an inducible promoter.

5. A method as claimed in any one of the preceding claims, wherein
(A) the nucleotide sequence encoding a plant-hormone biosynthetic polypeptide and the second terminator element are downstream of the first promoter, wherein the first promoter is capable of driving expression of the plant-hormone biosynthetic polypeptide; and/or
(B) the Transformation Cassette comprises:
(a) a first promoter which is operable in said plant cell,
(b) an Excision Cassette comprising:
(b1) a first site-specific recombination element,
(b3) a nucleotide sequence encoding a plant-hormone biosynthetic polypeptide, wherein the plant-hormone biosynthetic polypeptide is IPT (isopentenyl transferase),
(b4) a second terminator element,
(b5) a second site-specific recombination element,
wherein the first and second site-specific recombination element are capable of being recognised by a recombinase;
(c) one or more transgenes,
(d) a first terminator element,
operably linked in the order specified above in a 5'-3' direction.

6. A method as claimed in any one of claims 1 to 4 or claim 5, part (A), wherein the Excision Cassette is in the reverse orientation compared to the first promoter, transgene(s) and first terminator element, and the Excision Cassette comprises a second promoter which is capable of driving the expression of the nucleotide sequence encoding the plant-hormone biosynthetic polypeptide,
preferably wherein the Transformation Cassette comprises:
(a) a first promoter which is operable in said plant cell,
(b) an Excision Cassette,
(c) one or more transgenes,
(d) a first terminator element,
wherein (a), (b), (c) and (d) are operably linked in the order specified above in a 5'-3' direction,
wherein the Excision Cassette comprises:
(b1) a first site-specific recombination element,
(b2) a second promoter,
(b3) a nucleotide sequence encoding a plant-hormone biosynthetic polypeptide, wherein the plant-hormone biosynthetic polypeptide is IPT (isopentenyl transferase),
(b4) a second terminator element,
(b5) a second site-specific recombination element,
wherein the first and second site-specific recombination elements are capable of being recognised by a recombinase, and
wherein the parts (b1)-(b5) of the Excision Cassette are operably linked and wherein the Excision Cassette is in reverse orientation compared to (a), (c) and (d).

7. A method as claimed in claim 1 wherein the first and second site-specific recombination elements are lox elements and the recombinase is Cre, additionally comprising the steps (ii) selecting for transformed plant cells on media which is lacking cytokinin; and (iii) expressing a Cre recombinase in the plant cell at a level which results in excision of the Excision Cassette from the plastid genome.

8. A method as claimed in any one of the preceding claims, wherein
(A) the Transformation Cassette additionally comprises a nucleotide sequence encoding a polypeptide which confers resistance to an antibiotic, preferably wherein the antibiotic is spectinomycin; and/or
(B) wherein the plant is a monocot or dicot, preferably wherein the plant is selected from the group consisting of cereals (rice, wheat, barley, oats, sorghum, corn), legumes (alfalfa, lentils, peanut, pea, soybean), oil crops (palm, sunflower, coconut, canola, olive), cash crops (cotton, sugar cane, cassava), vegetable crops (potato, tomato, carrot, sweet potato, sugar-beet, squash, cucumber, lettuce, broccoli, cauliflower, snap bean, cabbage, celery, onion, garlic), fruits/trees and nuts (banana, grape cantaloupe, muskmelon, watermelon, strawberry, orange, apple, mango, avocado, peach, grapefruit, pineapple, maple, almond), beverages (coffee, tea, cocoa), and timber trees (oak, black walnut, sycamore), more preferably wherein the plant is tobacco or lettuce.

9. A method as claimed in any one of the preceding claims, wherein
(A) the plant cells are individual cells, groups of cells, in dissociated form or undissociated form, or as part of a plant tissue or plant part; and/or
(B) wherein the plant cells are present in plant leaves; and/or
(C) wherein the plastid is selected from chloroplasts, leucoplasts, amyloplasts, etioplasts, chromoplasts, elaioplasts and gerontoplasts; and/or
(D) wherein the plastid is a green plastid; and/or
(E) wherein the plastid is a chloroplast; and/or
(F) wherein the first and/or second promoter is a PsbA, RbcL or Prrn promoter; and/or
(G) wherein the first and/or second terminator is a rrn, psbA, rbcL or T7 terminator.

10. A transformed plant cell obtained by or obtainable by a method as claimed in any one of claims 1 to 9.

11. A method of producing a transgene product, comprising a method as defined in any one of the preceding claims, and additionally comprising the step of purifying or isolating, and optionally packaging, the transgene product.

12. A genetic construct comprising first and second homologous recombination elements flanking a Transformation Cassette, wherein the nucleotide sequences of the first and second homologous recombination elements are selected such that the Transformation Cassette is specifically targeted to one or more selected plastids and wherein the first and second homologous recombination elements are capable of directing the integration of the Transformation Cassette into the genome of the one or more selected plastids which are present in a plant cell,
wherein the Transformation Cassette comprises:
(a) a first promoter which is operable in a plant cell,
(b) an Excision Cassette,
(c) one or more transgenes,
(d) a first terminator element,
wherein the Excision Cassette comprises:
(b1) a first site-specific recombination element,
(b2) an optional second promoter
(b3) a nucleotide sequence encoding a plant-hormone biosynthetic polypeptide, wherein the plant-hormone biosynthetic polypeptide is IPT (isopentenyl transferase),
(b4) a second terminator element,
(b5) a second site-specific recombination element,
wherein the first and second site-specific recombination elements are capable of being recognised by a recombinase, preferably
(A) wherein the first and second site-specific recombination elements are lox sites; and/or
(B) wherein the recombinase is a Cre recombinase.

13. A plant cell, a plant or a plant seed comprising a genetic construct as claimed in claim 12.

## Patentansprüche

1. Verfahren zum Erzeugen einer transformierten Pflanzenzelle, wobei das Verfahren den Schritt umfasst:
(i) Transformieren der Pflanzenzelle mit einem genetischen Konstrukt,
wobei das genetische Konstrukt erste und zweite homologe Rekombinationselemente umfasst, die eine Transformationskassette flankieren, wobei die Nukleotidsequenzen der ersten und zweiten homologen Rekombinationselemente so ausgewählt sind, dass die Transformationskassette spezifisch zu ein oder mehreren ausgewählten Plastiden geführt wird, und wobei die ersten und zweiten homologen Rekombinationselemente in der Lage sind, die Integration der Transformationskassette in das Genom der ein oder mehreren ausgewählten Plastide, die in der Pflanzenzelle vorliegen, zu steuern,
wobei die Transformationskassette umfasst:
(a) einen ersten Promoter, welcher in der Pflanzenzelle operabel ist,
(b) eine Excisionskassette,
(c) ein oder mehrere Transgene,
(d) ein erstes Terminatorelement,
wobei die Excisionskassette umfasst:
(b1) ein erstes ortsspezifisches Rekombinationselement,
(b2) einen optionalen zweiten Promoter,
(b3) eine Nukleotidsequenz, die ein pflanzenhormonelles biosynthetisches Polypeptid kodiert, wobei das pflanzenhormonelle biosynthetische Polypeptid IPT (Isopentenyltransferase) ist,
(b4) ein zweites Terminatorelement,
(b5) ein zweites ortsspezifisches Rekombinationselement,
wobei die ersten und zweiten ortsspezifischen Rekombinationselemente von einer Rekombinase erkannt werden können, wobei das Verfahren zusätzlich den Schritt umfasst:
(ii)Selektieren von transformierten Pflanzenzellen auf Medium, welchem Cytokinin fehlt.

2. Verfahren nach Anspruch 1, wobei das Verfahren zusätzlich den Schritt umfasst:
(iii) Exprimieren einer Rekombinase in der Pflanzenzelle, wobei die Rekombinase eine ist, die die ersten und zweiten ortsspezifischen Rekombinationselemente erkennt, und gegebenenfalls Regenerieren einer Pflanze aus der transformierten Pflanzenzelle durch Hinzufügen von (A) Cytokinin und Auxin oder (B) Auxin.

3. Verfahren nach einem der vorherigen Ansprüche, wobei:
(A) die ersten und zweiten ortsspezifischen Rekombinationselemente lox-Stellen sind; und/oder
(B) die Rekombinase eine Cre-Rekombinase ist; und/oder
(C) wenigstens eines der ein oder mehreren Transgene einen Antikörper, ein Antibiotikum, ein Herbizid, ein Vakzinantigen, ein Enzym, einen Enzyminhibitor oder ein designtes Peptid kodiert; und/oder
(D) die Nukleotidsequenzen der homologen Rekombinationselemente so ausgewählt sind, dass keine oder im Wesentlichen keine Transformationskassetten in das Nukleargenom der Pflanze integriert werden.

4. Verfahren nach Anspruch 2 oder Anspruch 3, wobei
(A) Schritt (iii) Induzieren der Expression einer Rekombinase in der Pflanzenzelle durch einen induzierbaren Promoter operabel verknüpft mit einer Nukleotidsequenz, die eine Rekombinase kodiert, welche in der Pflanzenzelle vorliegt, wobei die Rekombinase die ersten und zweiten ortsspezifischen Rekombinationselemente erkennt, umfasst; oder
(B) Schritt (iii) Transformieren der Pflanzenzelle mit einem Rekombinasevektor, welcher einen Promoter operabel verknüpft mit einer Nukleotidsequenz, welche eine Rekombinase kodiert, umfasst, entweder vor Schritt (i), gleichzeitig mit Schritt (i) oder nach Schritt (i) oder vor Schritt (ii), gleichzeitig mit Schritt (ii) oder nach Schritt (ii), umfasst, bevorzugt wobei der Rekombinasevektor einen Operator operabel verknüpft mit einer Nukleotidsequenz umfasst, welche ein Plastid-ansteuerndes Transitpeptid und eine Rekombinase kodiert, mehr bevorzugt, wobei das Plastid-ansteuernde Transitpeptid eines ist, welches in der Lage ist, die Rekombinase zu einem Chloroplasten zu führen, gegebenenfalls wobei der Promoter ein induzierbarer Promoter ist.

5. Verfahren nach einem der vorherigen Ansprüche, wobei
(A) die Nukleotidsequenz kodierend ein pflanzenhormonelles biosynthetisches Polypeptid und das zweite Terminatorelement sich stromabwärts von dem ersten Promoter befinden, wobei der erste Promoter in der Lage ist, die Expression des pflanzenhormonellen biosynthetischen Polypeptids zu steuern; und/oder
(B) die Transformationskassette umfasst:
(a) einen ersten Promoter, welcher in der Pflanzenzelle operabel ist,
(b) eine Excisionskassette umfassend:
(b1) ein erstes ortsspezifisches Rekombinationselement,
(b3) eine Nukleotidsequenz, die ein pflanzenhormonelles biosynthetisches Polypeptid kodiert, wobei das pflanzenhormonelle biosynthetische Polypeptid IPT (Isopentenyltransferase) ist,
(b4) ein zweites Terminatorelement,
(b5) ein zweites ortsspezifisches Rekombinationselement,
wobei die ersten und zweiten ortsspezifischen Rekombinationselemente von einer Rekombinase erkannt werden können;
(c) ein oder mehrere Transgene,
(d) ein erstes Terminatorelement,
operabel verknüpft in der oben angegebenen Reihenfolge in einer 5'-3'-Richtung.

6. Verfahren nach einem der Ansprüche 1 bis 4 oder Anspruch 5, Teil (A), wobei die Excisionskassette verglichen mit dem ersten Promoter, Transgen(en) und dem ersten Terminatorelement sich in umgekehrter Reihenfolge befindet, und die Excisionskassette einen zweiten Promoter umfasst, welcher in der Lage ist, die Expression der Nukleotidsequenz zu steuern, die das pflanzenhormonelle biosynthetische Polypeptid kodiert,
bevorzugt wobei die Transformationskassette umfasst:
(a)einen ersten Promoter, welcher in der Pflanzenzelle operabel ist,
(b)eine Excisionskassette,
(c)ein oder mehrere Transgene,
(d)ein erstes Terminatorelement,
wobei (a), (b), (c) und (d) in der oben angegebenen Reihenfolge in einer 5'-3'-Richtung operabel verknüpft sind,
wobei die Excisionskassette umfasst:
(b1) ein erstes ortsspezifisches Rekombinationselement,
(b2) einen optionalen zweiten Promoter,
(b3) eine Nukleotidsequenz, die ein pflanzenhormonelles biosynthetisches Polypeptid kodiert, wobei das pflanzenhormonelle biosynthetische Polypeptid IPT (Isopentenyltransferase) ist,
(b4) ein zweites Terminatorelement,
(b5) ein zweites ortsspezifisches Rekombinationselement,
die ersten und zweiten ortsspezifischen Rekombinationselemente von der Rekombinase erkannt werden können und
wobei die Teile (b1)-(b5) der Excisionskassette operabel verknüpft sind und wobei sich die Excisionskassette verglichen mit (a), (c) und (d) in umgekehrter Orientierung befindet.

7. Verfahren nach Anspruch 1, wobei die ersten und zweiten ortsspezifischen Rekombinationselemente lox-Elemente sind und die Rekombinase Cre ist, weiterhin umfassend die Schritte (ii) Selektieren von transformierten Zellen auf Medium, welchem Cytokinin fehlt; und (iii) Exprimieren einer Cre-Rekombinase in der Planzenzelle auf einem Niveau, welches zu einer Excision der Excisionskassette aus dem Plastidgenom führt.

8. Verfahren nach einem der vorherigen Ansprüche, wobei
(A) die Transformationskassette weiterhin eine Nukleotidsequenz umfasst, die ein Polypeptid kodiert, welches eine Resistenz gegen ein Antibiotikum verleiht, bevorzugt wobei das Antibiotikum Spectinomycin ist; und/oder
(B) wobei die Pflanze eine Monokotyledone oder Dikotyle ist, bevorzugt wobei die Pflanze ausgewählt ist aus der Gruppe bestehend aus Getreide (Reis, Weizen, Gerste, Hafer, Hirse, Mais), Hülsenfrüchten (Alfalfa, Linsen, Erdnuss, Erbse, Sojabohne), Ölfrüchten (Palme, Sonnenblume, Kokosnuss, Canola, Olive), Zwischenfrüchten (Baumwolle, Zuckerrohr, Kassave), Gemüsepflanzen (Kartoffel, Tomate, Karotte, Süßkartoffel, Zuckerrübe, Kürbis, Gurke, Salat, Brokkoli, Blumenkohl, Brechbohne, Kohl, Sellerie, Zwiebel, Knoblauch), Früchte/Bäume und Nüsse (Banane, Traube, Cantaloupe-Melone, Zuckermelone, Wassermelone, Erdbeere, Orange, Apfel, Mango, Avocado, Pfirsich, Grapefruit, Ananas, Ahorn, Mandel), Getränke (Kaffee, Tee, Kakao) und Nutzhölzer (Eiche, Schwarznuss, Platane), mehr bevorzugt, wobei die Pflanze Tabak oder Salat ist.

9. Verfahren nach einem der vorherigen Ansprüche, wobei
(A) die Pflanzenzellen als Einzelzellen, Zellgruppen, in dissoziierter Form oder undissoziierter Form, oder als Teil eines Pflanzengewebes oder Pflanzenteils vorliegen; und/oder
(B) wobei die Pflanzenzellen in Pflanzenblättern vorliegen; und/oder
(C) wobei das Plastid ausgewählt ist aus Chloroplasten, Leukoplasten, Amyloplasten, Etioplasten, Chromoplasten, Elaioplasten und Gerontoplasten; und/oder
(D) wobei das Plastid ein grünes Plastid ist; und/oder
(E) wobei das Plastid ein Chloroplast ist; und/oder
(F) wobei der erste und/oder zweite Promoter ein PsbA-, RbcL- oder Prrn-Promoter ist; und/oder
(G) wobei der erste und/oder zweite Terminator ein rrn-, psbA-, rbcL-oder T7-Terminator ist.

10. Transformierte Pflanzenzelle erhalten oder erhältlich mittels eines Verfahrens nach einem der Ansprüche 1 bis 9.

11. Verfahren zum Erzeugen eines transgenen Produkts umfassend ein Verfahren nach einem der vorherigen Ansprüche und weiterhin umfassend den Schritt des Aufreinigens oder Isolierens, und gegebenenfalls des Verpackens, des transgenen Produkts.

12. Genetisches Konstrukt umfassend erste und zweite homologe Rekombinationselemente, die eine Transformationskassette flankieren, wobei die Nukleotidsequenzen der ersten und zweiten homologen Rekombinationselemente so ausgewählt sind, dass die Transformationskassette spezifisch zu ein oder mehreren ausgewählten Plastiden geführt wird, und wobei die ersten und zweiten homologen Rekombinationselemente in der Lage sind, die Integration der Transformationskassette in das Genom der ein oder mehreren ausgewählten Plastide, die in der Pflanzenzelle vorliegen, zu steuern,
wobei die Transformationskassette umfasst:
(a) einen ersten Promoter, welcher in der Pflanzenzelle operabel ist,
(b) eine Excisionskassette,
(c) ein oder mehrere Transgene,
(d) ein erstes Terminatorelement,
wobei die Excisionskassette umfasst:
(b1) ein erstes ortsspezifisches Rekombinationselement,
(b2) einen optionalen zweiten Promoter,
(b3) eine Nukleotidsequenz, die ein pflanzenhormonelles biosynthetisches Polypeptid kodiert, wobei das pflanzenhormonelle biosynthetische Polypeptid IPT (Isopentenyltransferase) ist,
(b4) ein zweites Terminatorelement,
(b5) ein zweites ortsspezifisches Rekombinationselement,
wobei die ersten und zweiten ortsspezifischen Rekombinationselemente von einer Rekombinase erkannt werden können, bevorzugt
(A) wobei die ersten und zweiten ortsspezifischen Rekombinationselemente lox-Stellen sind; und/oder
(B) wobei die Rekombinase eine Cre-Rekombinase ist.

13. Pflanzenzelle, Pflanze oder Pflanzensamen umfassend ein genetisches Konstrukt nach Anspruch 12.

## Revendications

1. Procédé de production d'une cellule végétale transformée, le procédé comprenant les étapes consistant à :
(i) transformer la cellule végétale avec une construction génétique,
dans lequel la construction génétique comprend un premier et un deuxième élément de recombinaison homologue flanquant une cassette de transformation, dans lequel les séquences nucléotidiques des premier et deuxième éléments de recombinaison homologue sont choisies de sorte que la cassette de transformation soit spécifiquement ciblée sur un ou plusieurs plastides choisis et dans lequel les premier et deuxième éléments de recombinaison homologue dirigent l'intégration de la cassette de transformation dans le génome des un ou plusieurs plastides choisis qui sont présents dans la cellule végétale,
dans lequel la cassette de transformation comprend :
(a) un premier promoteur qui peut opérer dans ladite cellule végétale,
(b) une cassette d'excision,
(c) un ou plusieurs transgènes,
(d) un premier élément terminateur,
dans lequel la cassette d'excision comprend :
(b1) un premier élément de recombinaison spécifique au site,
(b2) un deuxième promoteur facultatif,
(b3) une séquence nucléotidique codant pour un polypeptide biosynthétique d'hormone végétale, dans lequel le polypeptide biosynthétique d'hormone végétale est l'IPT (isopentényl transférase),
(b4) un deuxième élément terminateur,
(b5) un deuxième élément de recombinaison spécifique au site,
dans lequel les premier et deuxième éléments de recombinaison spécifiques au site sont capables d'être reconnus par une recombinase, dans lequel le procédé comprend en outre l'étape consistant à :
(ii) choisir pour les cellules végétales transformées un milieu qui manque de cytokinine.

2. Procédé selon la revendication 1, dans lequel le procédé comprend en plus l'étape consistant à :
(iii) exprimer une recombinase dans la cellule végétale, dans lequel la recombinase en est une qui reconnaît les premier et deuxième éléments de recombinaison spécifiques au site, et régénérer éventuellement une plante à partir de la cellule végétale transformée en ajoutant (A) de la cytokinine et de l'auxine ou (B) de l'auxine.

3. Procédé selon l'une quelconque des revendications précédentes, dans lequel :
(A) les premier et deuxième éléments de recombinaison spécifiques au site sont des sites lox ; et/ou
(B) la recombinase est une recombinase Cre ; et/ou
(C) au moins l'un des un ou plusieurs transgènes code pour un anticorps, un antibiotique, un herbicide, un antigène de vaccin, un enzyme, un inhibiteur d'enzyme ou un peptide de conception ; et/ou
(D) les séquences nucléotidiques des éléments de recombinaison homologue sont choisis de sorte qu'aucune ou essentiellement aucune cassette de transformation ne s'intègre au génome nucléaire de la plante.

4. Procédé selon la revendication 2 ou la revendication 3, dans lequel :
(A) l'étape (iii) comprend l'induction de l'expression d'une recombinase dans la cellule végétale à partir d'un promoteur inductible lié en service à une séquence nucléotidique codant pour une recombinase qui est présente dans la cellule végétale, dans lequel la recombinase reconnaît les premier et deuxième éléments de recombinaison spécifiques au site ; ou
(B) l'étape (iii) comprend la transformation de la cellule végétale avec un vecteur de recombinase qui comprend un promoteur lié en service à une séquence nucléotidique codant pour une recombinase avant l'étape (i), simultanément avec l'étape (i) ou après l'étape (i) ou avant l'étape (ii), simultanément avec l'étape (ii) ou après l'étape (ii), de préférence, dans lequel le vecteur de recombinase comprend un promoteur lié en service à une séquence nucléotidique codant pour un peptide de transit ciblant les plastides et une recombinase, mieux encore dans lequel le peptide de transit ciblant les plastides est un peptide qui est capable de cibler la recombinase sur un chloroplaste, éventuellement lorsque le promoteur est un promoteur inductible.

5. Procédé selon l'une quelconque des revendications précédentes, dans lequel :
(A) la séquence nucléotidique codant pour un polypeptide biosynthétique d'hormone végétale et le deuxième élément terminateur sont en aval du premier promoteur, dans lequel le premier promoteur est capable d'entraîner l'expression du polypeptide biosynthétique d'hormone végétale ; et/ou
(B) la cassette de transformation comprend :
(a) un premier promoteur qui peut opérer dans ladite cellule végétale,
(b) une cassette d'excision comprenant :
(b1) un premier élément de recombinaison spécifique au site,
(b3) une séquence nucléotidique codant pour un polypeptide biosynthétique d'hormone végétale, dans lequel le polypeptide biosynthétique d'hormone végétale est l'IPT (isopentényl transférase),
(b4) un deuxième élément terminateur,
(b5) un deuxième élément de recombinaison spécifique au site,
dans lequel les premier et deuxième éléments de recombinaison spécifiques au site sont capables d'être reconnus par une recombinase ;
(c) un ou plusieurs transgènes,
(d) un premier élément terminateur,
liés en service dans l'ordre spécifié ci-dessus dans une direction 5'-3'.

6. Procédé selon l'une quelconque des revendications 1 à 4 ou la revendication 5, partie (A), dans lequel la cassette d'excision se trouve dans l'orientation inverse en comparaison du premier promoteur, du ou des transgènes et du premier élément terminateur, et la cassette d'excision comprend un deuxième promoteur qui est à même d'entraîner l'expression de la séquence nucléotidique codant pour le polypeptide biosynthétique d'hormone végétale,
de préférence, dans lequel la cassette de transformation comprend :
(a) un premier promoteur qui peut opérer dans ladite cellule végétale,
(b) une cassette d'excision,
(c) un ou plusieurs transgènes,
(d) un premier élément terminateur,
dans lequel (a), (b), (c) et (d) sont liés en service dans l'ordre spécifié ci-dessus dans la direction 5'-3'
dans lequel la cassette d'excision comprend :
(b1) un premier élément de recombinaison spécifique au site,
(b2) un deuxième promoteur,
(b3) une séquence nucléotidique codant pour un polypeptide biosynthétique d'hormone végétale, dans lequel le polypeptide biosynthétique d'hormone végétale est l'IPT (isopentényl transférase),
(b4) un deuxième élément terminateur,
(b5) un deuxième élément de recombinaison spécifique au site,
dans lequel les premier et deuxième éléments de recombinaison spécifiques au site sont capables d'être reconnus par une recombinase, et
dans lequel les parties (b1)-(b5) de la cassette d'excision sont liées en service et dans lequel la cassette d'excision est dans l'orientation inverse en comparaison de (a), (c) et (d).

7. Procédé selon la revendication 1, dans lequel les premier et deuxième éléments de recombinaison spécifiques au site sont des éléments lox et la recombinase est la Cre, comprenant en outre les étapes visant à (ii) choisir pour les cellules végétales transformées un milieu qui manque de cytokinine ; et (iii) exprimer une recombinase Cre dans la cellule végétale à un niveau qui entraîne une excision de la cassette d'excision du génome de plastide.

8. Procédé selon l'une quelconque des revendications précédentes, dans lequel :
(A) la cassette de transformation comprend en outre une séquence nucléotidique codant pour un polypeptide qui confère une résistance à un antibiotique, de préférence dans lequel l'antibiotique est la spectinomycine ; et/ou
(B) dans lequel la plante est une monocotylédone ou une dicotylédone, de préférence dans lequel la plante est choisie dans le groupe constitué des céréales (riz, blé, orge, avoine, sorgho, maïs), des légumineuses (luzerne, lentille, arachide, pois, soja), des oléagineux (palme, tournesol, coco, canola, olive), des cultures de rapport (coton, canne à sucre, manioc), des cultures de légumes (pomme de terre, tomate, carotte, patate douce, betterave sucrière, courge, concombre, laitue, brocoli, choux fleur, haricot vert, chou, céleri, ognon, ail), des fruits, arbres et noix (banane, raisin cantaloup, melon musqué, pastèque, fraise, orange, pomme, mangue, avocat, pêche, pamplemousse, ananas, érable, amande), des boissons (café, thé, cacao) et des arbres de construction (chêne, noyer noir, sycomore), mieux encore dans lequel la plante est le tabac ou la laitue.

9. Procédé selon l'une quelconque des revendications précédentes, dans lequel :
(A) les cellules végétales sont des cellules individuelles, des groupes de cellules, sous forme dissociée ou non dissociée, ou une partie d'un tissu végétal ou une partie de plante ; et/ou
(B) dans lequel les cellules végétales sont présentes dans les feuilles des plantes ; et/ou
(C) dans lequel le plastide est choisi parmi les chloroplastes, les leucoplastes, les amyloplastes, les étioplastes, les chromoplastes, les élaioplastes et les gérontoplastes ; et/ou
(D) dans lequel le plastide est un plastide vert ; et/ou
(E) dans lequel le plastide est un chloroplaste ; et/ou
(F) dans lequel le premier et/ou le deuxième promoteur est ou sont un promoteur PsbA, RbcL ou Prrn ; et/ou
(G) dans lequel le premier et/ou le deuxième terminateur et ou sont un terminateur rrn, psbA, rbcL ou T7.

10. Cellule végétale transformée obtenue ou susceptible d'être obtenue par un procédé selon l'une quelconque des revendications 1 à 9.

11. Procédé de production d'un produit transgénique, comprenant un procédé tel que défini dans l'une quelconque des revendications précédentes, et comprenant en outre l'étape de purification ou d'isolement et éventuellement d'emballage du produit transgénique.

12. Construction génétique comprenant un premier et un deuxième élément de recombinaison homologue flanquant une cassette de transformation, dans laquelle les séquences nucléotidiques des premier et deuxième éléments de recombinaison homologue sont choisis de sorte que la cassette de transformation soit spécifiquement ciblée sur un ou plusieurs plastides choisis et dans laquelle les premier et deuxième éléments de recombinaison homologue sont capables de diriger l'intégration de la cassette de transformation dans le génome des un ou plusieurs plastides choisis qui sont présents dans une cellule végétale,
dans laquelle la cassette de transformation comprend :
(a) un premier promoteur qui est à même d'opérer dans une cellule végétale,
(b) une cassette d'excision,
(c) un ou plusieurs transgènes,
(d) un premier élément terminateur,
dans lequel la cassette d'excision comprend :
(b1) un premier élément de recombinaison spécifique au site,
(b2) un deuxième promoteur facultatif,
(b3) une séquence nucléotidique codant pour un polypeptide biosynthétique d'hormone végétale, dans laquelle le polypeptide biosynthétique d'hormone végétale est l'IPT (isopentényl transférase),
(b4) un deuxième élément terminateur,
(b5) un deuxième élément de recombinaison spécifique au site,
dans laquelle les premier et deuxième éléments de recombinaison spécifiques au site sont capables d'être reconnus par une recombinase, de préférence
(A) dans laquelle les premier et deuxième éléments de recombinaison spécifiques au site sont des sites lox ; et/ou
(B) dans laquelle la recombinase est une recombinase Cre.

13. Cellule végétale, plante ou semence de plante comprenant une construction génétique selon la revendication 12.
